# EUROPEAN PATENT APPLICATION

(11) **EP 4 129 325 A1**
(43) Date of publication of application: **08.02.2023**
(21) Application number: 20927430.7
(22) Date of filing: 16.06.2020
(51) Int. Cl.: A61K 39/00, A61K 9/16, A61P 35/00

(54) **WHOLE-CELL CONSTITUENT TRANSPORT SYSTEM AND APPLICATION THEREOF**

(30) Priority: 26.03.2020 CN 202010223563
(71) Applicant: Suzhou Ersheng Biopharmaceutical Co., Ltd, Suzhou, Jiangsu 215127 (CN)
(72) Inventor: LIU, Mi, Suzhou, Jiangsu 215123 (CN)
(74) Representative: Greaves Brewster LLP
(86) International application number: PCT/CN2020/096302
(87) International publication number: WO 2021/189678

(57) **Abstract**

A transport system which utilizes nanoscale size or microscale size particles to deliver whole-cell constituent water soluble components and water insoluble components, and is used in an application for preparing a vaccine that prevents and treats cancer. The whole-cell constituent transport system consists of nanoscale size or microscale size particles and whole-cell constituents loaded onto said particles, the whole-cell constituents being whole-cell water soluble components and water insoluble components among cells or tissues. Among cell constituents, mutated proteins or polypeptides produced due to cancer are loaded onto the nanoparticles or micronparticles. Among whole-cell constituents, these substances, which possess immunogenicity and which are produced due to illness and mutation, can be used for the treatment and prevention of cancer, and can be used to prepare a vaccine for the prevention and/or treatment of cancer.

## Description

This application claims the priority of the Chinese patent application filed in China National Intellectual Property Administration on Mar. 26, 2020, with the application number of 202010223563.2, entitled "WHOLE-CELL COMPONENTS DELIVERY SYSTEM AND APPLICATION THEREOF", the entire contents of which is incorporated herein by reference.

### TECHNICAL FIELD

The present disclosure relates to the field of immunotherapy, specifically relates to a whole-cell components delivery system and application thereof, and particularly relates to a whole-cell components delivery system and its application in preparing preventive and therapeutic vaccines for cancer.

### BACKGROUND

Immunity is a physiological function of the human body that allows the body to recognize "self" and "non-self" components and thus destroy and reject antigenic substances (such as viruses and bacteria) that enter the body, or damaged cells and tumor cells produced by the body itself, in order to maintain the body health. Immunological technology has developed extremely rapidly in recent years, particularly in the field of cancer immunotherapy. With the increasing understanding of cancer, it has been discovered that the body's immune system and various types of immune cells play key roles in inhibiting the development and progression of cancer.

In recent years, therapies such as PD-1 antibodies and CAR-T have been approved to be used in clinic and showed good clinical efficacy. However, such immunotherapy methods also have some significant limitations. Currently, cancer immunotherapies based on PD-1 antibody and CAR-T are only effective for some specific patients. Taking CAR-T as an example, as the B cell surface-specific targets such as CD19, CD20 and CD22 used are hard to find or almost absent in solid tumors, therapies such as CAR-T are currently only applicable to the treatment of hematological tumors.

In order to improve the treatment of solid tumors, scientists in the United States and Germany have adopted new technologies to analyze and identify tumor-specific or tumor-related antigenic peptides from tumor cells of cancer patients, and then artificially synthesize them *in vitro* to prepare cancer vaccines for cancer treatment. The technology has shown some efficacy in clinical trials of cancer patients. However, such methods are time-consuming, labor-intensive, and expensive. Moreover, the methods used are only to extract and analyze the difference between cancer cells and normal cells from the water-soluble components of cancer cells, resulting in finding limited antigenic peptides with good water-solubility, which greatly limit the application of such methods. However, many antigenic proteins or peptides with strong immunogenicity in the real environment of the human body are insoluble in pure water and need to exist in the help of binding with proteins, adsorbing or locating on the membrane or membrane surface in the body. Therefore, this portion of the water-insoluble proteins and peptides that are insoluble in pure water is very important and critical. However, there has been no method to use the whole-cell components of cancer cells as a vaccine for the prevention and treatment of cancer currently.

### SUMMARY

In view of this, for the problems existing in the prior art, the objective of the present invention is to provide a whole-cell components delivery system and application thereof, wherein the whole-cell components contains both water-soluble components and water-insoluble components insoluble in pure water or a water solution without a solubilizer.

In order to achieve the objective of the present invention, the technical solutions used in the present invention are as follows:

A whole-cell components delivery system consisting of a nano-sized particle (nanoparticle) or micron-sized particle (micronparticle) and whole-cell components loaded on the particle, wherein the whole-cell components are water-soluble components and water-insoluble components of a whole cell in a cell or tissue.

In the delivery system described in the present invention, the forms to load are the water-soluble components and the water-insoluble components of the whole cell are separately or together loaded inside the particle, and/or, separately or together loaded on the surface of the particle. The forms include, but not limited to, loading the water-soluble components together inside the particle and on the surface of the particle, loading the water-insoluble components inside the particle and on the surface of the particle, loading the water-soluble components inside the particle and the water-insoluble components on the surface of the particle, loading the water-insoluble components inside the particle and the water-soluble components on the surface of the particle, loading the water-soluble components and the water-insoluble components inside the particles with the water-insoluble components being loaded on the surface of the particle alone, loading the water-soluble components and the water-insoluble components inside the particle with the water-soluble components being loaded on the surface of the particle alone, loading the water-soluble components inside the particle with the water-soluble components and the water-insoluble components being together loaded on the surface of the particle, loading the water-insoluble components inside the particle with the water-soluble components and the water-soluble components being together loaded on the surface of the particle, loading the water-soluble components and the water-insoluble components together inside the particle with the water-soluble components and the water-soluble components being together loaded on the surface of the particle.

In some embodiments, the inside and/or the surface of the particle further comprises an immune enhancing adjuvant in the delivery system.

The method of adding the immune enhancing adjuvant includes being loaded inside the nanoparticle or micronparticle, or on the surface of the nanoparticle or micronparticle, or inside the nanoparticle or micronparticle and on the surface of the nanoparticle or micronparticle together.

In the delivery system described in the present invention, the whole-cell components can be divided into two parts according to solubility in pure water or water solution without a solubilizer: the water-soluble components and the water-insoluble components. The water-soluble components are the original water-soluble portion that is soluble in pure water or water solution without a solubilizer. The water-insoluble components are the original water-insoluble portion that is insoluble in pure water, which are converted from being insoluble in pure water or water solution without a solubilizer to being soluble in water solution containing a solubilizer or organic solvent by a suitable solubilization method. Both the water-soluble portion and the water-insoluble portion of the whole-cell components can be dissolved in a solubilizing water solution containing a solubilizer or an organic solvent.

In the delivery system described in the present invention, the solubilizer is at least one of the solubilizers that can increase the solubility of proteins or peptides in a water solution; the organic solvent is an organic solvent that can dissolve proteins or peptides.

In the delivery system described in the present invention, the solubilizer include but is not limited to urea, guanidine hydrochloride, sodium deoxycholate, SDS, glycerol, alkaline solution with a pH more than 7, acidic solution with a pH less than 7, various protein degrading enzymes, albumin, lecithin, inorganic salts at high concentrations, Triton, DMSO, acetonitrile, ethanol, methanol, DMF, propanol, isopropanol, Tween, acetic acid, cholesterol, amino acids, glycosides, choline, Brij-35, Octaethylene glycol monododecyl ether, CHAPS, Digitonin, lauryldimethylamine oxide and IGEPAL^{®} CA-630. Those skilled in the art can understand that the water-insoluble components can also be converted from being insoluble in pure water to being soluble by other methods that can solubilize proteins and peptide fragments.

In the delivery system described in the present invention, the organic solvent include but not limited to DMSO, acetonitrile, ethanol, methanol, DMF, isopropanol, dichloromethane, and ethyl acetate. Those skilled in the art can understand that the organic solvent can also adopt other methods containing organic solvent that can solubilize protein and peptide fragments.

In the delivery system described in the present invention, the immune enhancing adjuvant includes but not limited to at least one of immune enhancers derived from microorganism, products of human or animal immune systems, intrinsic immune agonists, adaptive immune agonists, chemically synthesized medicaments, fungal polysaccharides and traditional Chinese medicines.

In the present invention, the immune enhancing adjuvant includes but not limited to at least one of pattern recognition receptor agonist, Bacille Calmette-Guérin (BCG) vaccine, BCG cell wall skeleton, residues frommethanol extraction of BCG, BCG cell wall acyl dipeptide, Mycobacterium phlei, thymosin, polyactin A, mineral oil, virus-like particles, immune enhanced reconstituted influenza virus bodies, cholera enterotoxin, saponin and derivatives thereof, Resiquimod, newborn bovine liver active peptides, miquimod, polysaccharides, curcumin, immune adjuvant CpG, immune adjuvant poly(I:C), immune adjuvant poly ICLC, Corynebacterium parvum, hemolytic streptococcus preparations, coenzyme Q10, levamisole, polyinosinic acid, interleukin, interferon, polyinosinic acid, polyadenylic acid, alum, aluminum phosphate, lanolin, vegetable oil, endotoxin, liposome adjuvants, GM-CSF, MF59, double-stranded RNA, double strand DNA, aluminum hydroxide, CAF01, active ingredients of ginseng and active ingredients of Astragalus membranaceus.

Those skilled in the art can understand that the immune enhancing adjuvant can also use other substances that can enhance the immune response.

In the delivery system described in the present invention, the nano-sized particle has a particle size of 1 nm to 1,000 nm. In some embodiments, the nano-sized particle has a particle size of 50 nm to 800 nm. In some embodiments, the nano-sized particle further has a particle size of 100 nm to 600 nm.

In the delivery system described in the present invention, the micron-sized particle has a particle size of 1 µm to 1,000 µm. In some embodiments, the micron-sized particle has a particle size of 1 µm to 100 µm. In some embodiments, the micron-sized particle has a particle size of 1 µm to 10 µm. In some embodiments, the micron-sized particle further has a particle size of 1 µm to 5 µm.

In the delivery system described in the present invention, the nano-sized particle and micron-sized particle are electrically neutral, negatively charged or positively charged on surface.

In the delivery system described in the present invention, the nano-sized or micro-sized particle is made of materials as organic synthetic polymer materials, natural polymer materials or inorganic materials.

Herein, the organic synthetic polymer materials are biocompatible or degradable polymer materials, include but not limited to PLGA, PLA, PGA, Poloxamer, PEG, PCL, PEI, PVA, PVP, PTMC, polyanhydride, PDON, PPDO, PMMA, polyamino acid, and synthetic peptides.

The natural polymer materials are biocompatible or degradable polymer materials, include but not limited to lecithin, cholesterol, starch, sugar, polypeptides, sodium alginate, albumin, collagen, gelatin, and cell membrane components.

The inorganic materials are materials without obvious biological toxicity, include but not limited to ferric oxide, iron oxide, calcium carbonate, and calcium phosphate.

The delivery system described in the present invention has a shape which is any shape commonly used, includes but not limited to spherical, ellipsoidal, barrel-shaped, polygonal, rod-shaped, sheet-shaped, linear, worm-shaped, square, triangular, butterfly-shaped and disc-shaped.

The whole-cell components delivery system described in the present invention can be prepared according to the developed preparation methods of nano-sized particles and micro-sized particles, including but not limited to common solvent evaporation method, dialysis method, extrusion method and hot-melt method. In some embodiments, the delivery system is prepared by a double emulsion method in the solvent evaporation method.

The whole-cell components delivery system described in the present invention can deliver the loaded whole-cell components to the relevant immune cells, activating and enhancing the killing effect of body immune system on cancer cells through the immunogenicity of the loaded composition. Therefore, the present invention also provides an application of the whole-cell components delivery system in preparing vaccines for preventing and/or treating cancer.

When preventing or treating diseases, the whole-cell components delivery system of the present invention can use simultaneously nanoparticles or micronparticles loaded with water-soluble components alone and nanoparticles or micronparticles loaded with water-insoluble components alone, nanoparticles or micronparticles loaded with water-soluble components alone, nanoparticles or micronparticles loaded with water-insoluble components alone, or nanoparticles or micronparticles loaded with water-soluble and water-insoluble components together.

According to the above technical solutions, the present invention provides a delivery system for delivering cellular water-soluble components and water-insoluble components using nano-sized or micro-sized particles, as well as applications in the preparation of vaccines for preventing and treating cancer. As the whole-cell components of the relevant cell or tissue is divided into two parts according to the solubility in pure water: the water-soluble portion that is soluble in pure water and the water-insoluble portion that is insoluble in pure water. And the water-soluble portion and the water-insoluble portion are both loaded in nanoparticles or micronparticles. Therefore, most of the mutated proteins or peptides produced by cancer in cellular components are loaded in nanoparticles or micronparticles. The water-soluble portion and the water-insoluble portion of the cell components cover all the components of the whole cell; the water-soluble portion and the water-insoluble portion of the cell components can also be dissolved by the water solution containing a solubilizer at the same time. Herein, the unmutated proteins, peptides and genes that have the same components with the normal cell components will not cause an immune response because of the immune tolerance generated during the development of the body immune system; and mutations in genes, proteins and peptides caused by cancer, etc. are immunogenic and can activate immune response because of lacking immune tolerance generated during the development of the body immune system. These immunogenic substances generated by mutations in disease in whole-cell components can be used for cancer treatment.

The whole-cell components delivery system described in the present invention can prepare vaccines for preventing and/or treating cancer. The cancer is any types of cancer including hematological tumors and solid tumors, including but not limited to endocrine system tumor, nervous system tumor, reproductive system tumor, digestive system tumor, respiratory system tumor, blood cancer, skin cancer, breast cancer, lung cancer, liver cancer, stomach cancer, pancreatic cancer, brain cancer, colon cancer, prostate cancer, rectal cancer, head and neck cancer, kidney cancer, bone cancer, nose cancer, bladder cancer, thyroid cancer, esophagus cancer, cervical cancer, ovarian cancer, uterine cancer, pelvic cancer, testicular cancer, penile cancer, lymphoma, tongue cancer, gum cancer, retinoblastoma and sarcoma, etc. When used as a cancer vaccine to prevent and treat cancer, the vaccine of the present invention can be administered multiple times before or after the occurrence of cancer to activate the body's immune system, thereby delaying the progression of cancer, treating cancer, preventing the occurrence of cancer, preventing cancer metastasis or recurrence.

### BRIEF DESCRIPTION OF THE DRAWINGS

In order to illustrate the embodiments of the present invention or the technical solutions in the prior art more clearly, brief introduction of the accompanying drawings required in the description of the embodiments or the prior art are as follows.

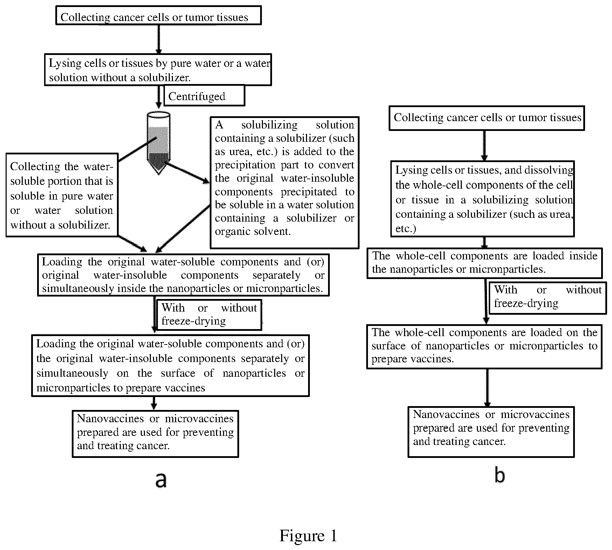

Figure 1 is the schematic diagram of the preparation process and application fields of vaccines described in the present invention; a: a schematic diagram of the collection of water-soluble components and water-insoluble components respectively and preparation of a nanovaccine or a microvaccine; b: schematic diagram of using a solubilizing solution containing a solubilizer to dissolve the whole-cell components and prepare a nanovaccine or a microvaccine.

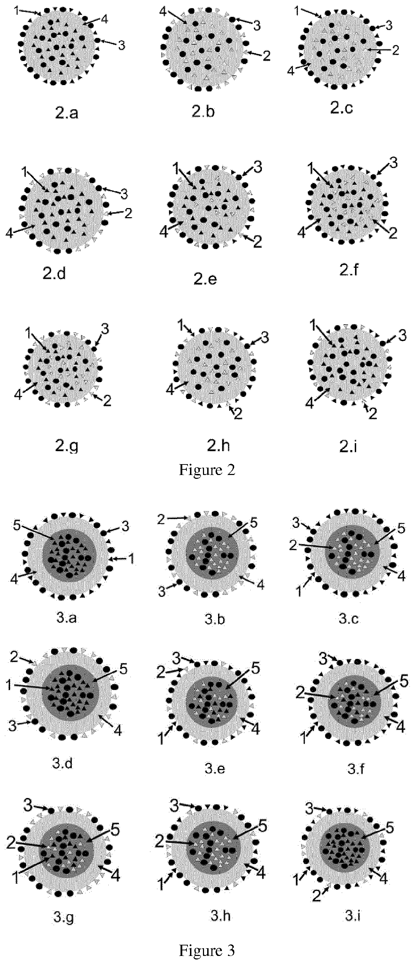

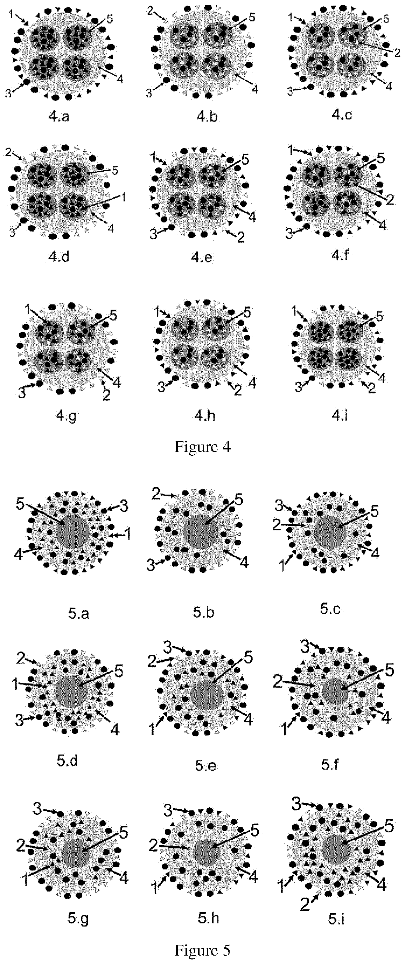

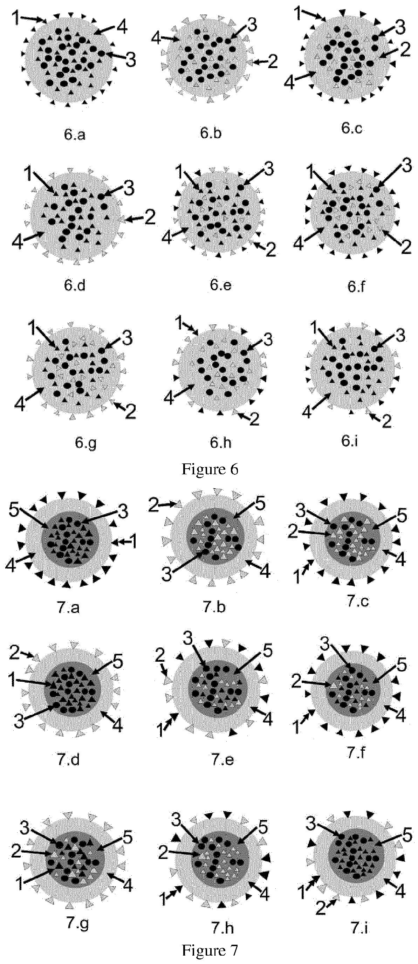

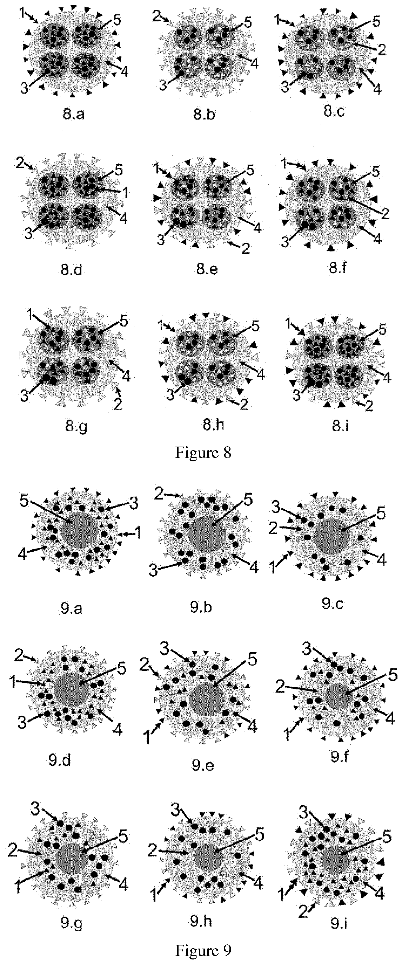

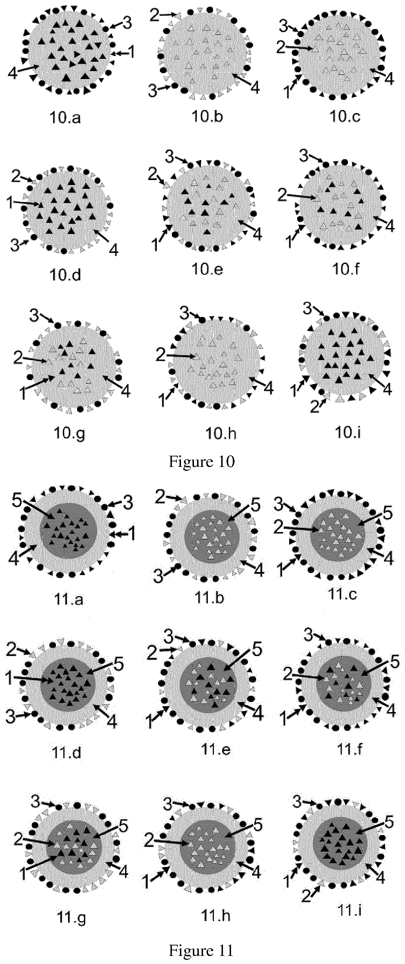

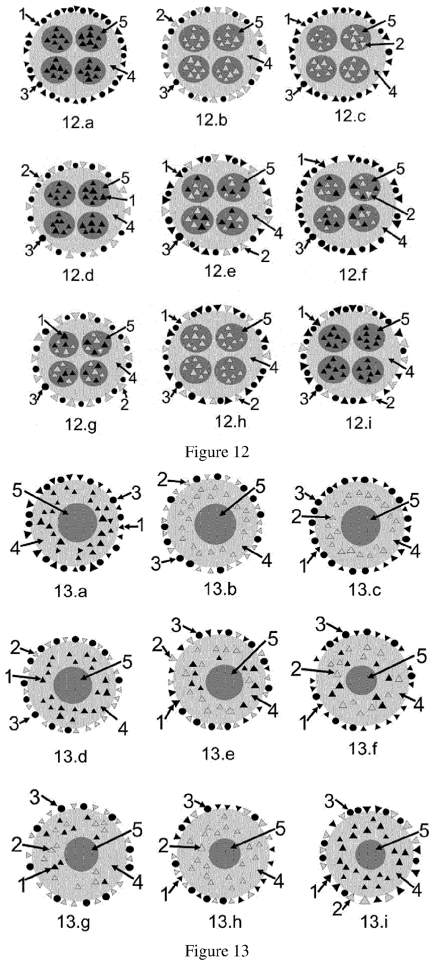

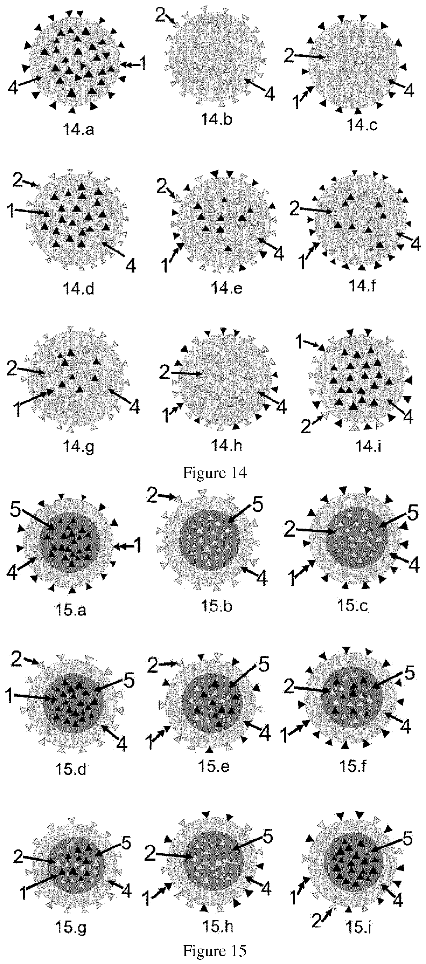

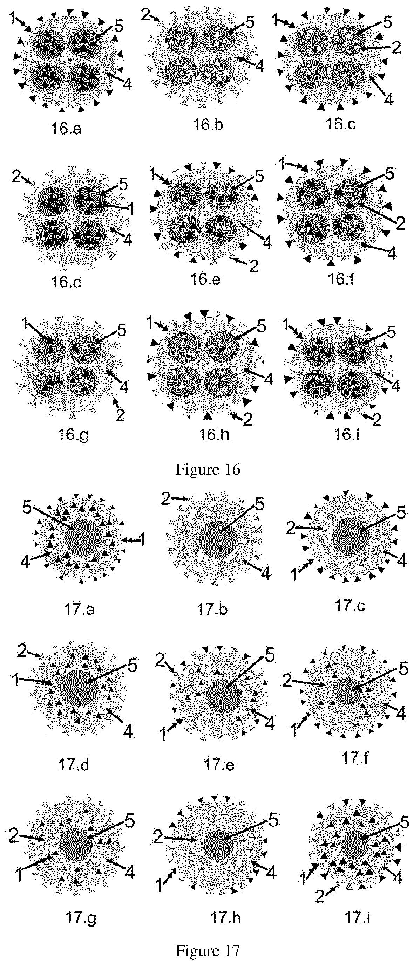

Figure 2 to Figure 17 are schematic diagrams of the structures of nano-sized particles or micro-sized particles loaded with water-soluble and water-insoluble cellular components, wherein 1, water-soluble components in a cells or tissue; 2, water-insoluble components in a cell or tissue; 3, immune enhancing adjuvant; 4, nanoparticles or micronparticles; 5, the inner core part of a nanoparticle; in Figure 2- Figure 5, both the surface and the interior of the nanoparticle or microparticle contain an immune enhancing adjuvant; in Figure. 6- Figure 9, the immune enhancing adjuvant is only distributed inside the nanoparticles or micronparticles; in Figure 10-Figure 13, only the outer surface of a nanoparticle or micronparticle contain an immune enhancing adjuvant; in Figure 14- Figure 17, the inside and outer surface of a nanoparticle or micronparticle has no immune enhancing adjuvant; in Figure 2, Figure 6, Figure 10 and Figure 14, when the water-soluble or water-insoluble components of a cell or tissue component loaded on the nanoparticle or micronparticle are distributed inside the nanoparticle or micronparticle, no obvious inner core is formed; in Figure 3, Figure 7, Figure 11 and Figure 15, when the water-soluble or water-insoluble components of the cell or tissue components loaded on the nanoparticle or micronparticle are distributed inside the nanoparticle or micronparticle, one inner core portion is formed, and the inner core can be generated during the preparation process or formed by the use of polymers or inorganic salts, etc.; in Figure 4, Figure 8, Figure 12 and Figure 16, when the water-soluble or water-insoluble components of the cell or tissue components loaded on the nanoparticle or micronparticle are distributed inside the nanoparticle or micronparticle, a plurality of inner core portions are formed , the inner core can be generated during the preparation process or formed by the use of polymers or inorganic salts, etc.; in Figure 5, Figure 9, Figure 13 and Figure 17, when the water-soluble or water-insoluble components of the cell or tissue components loaded on the nanoparticle or micronparticle are distributed inside the nanoparticle or micronparticle, they are located in the outer layer of the formed inner core; a: it is the water-soluble components of a cell or tissue components loaded both inside and on the surface of nanoparticles or micronparticles; b: it is the water-insoluble components of a cell or tissue components loaded both inside and on the surface of nanoparticles or micronparticles; c: it is the water-insoluble components of a cell or tissue components loaded inside of nanoparticles or micronparticles and it is the water-soluble components of a cell or tissue components loaded on the surface; d: it is the water-soluble components of a cell or tissue components loaded inside the nanoparticles or micronparticles, and it is the water-insoluble components of a cell or tissue components loaded on the surface; e: it is the water-soluble components and water-insoluble components of a cell or tissue components loaded together inside the nanoparticles or micronparticles, while water-soluble components and water-insoluble components of a cell or tissue components are also loaded on the surface of nanoparticles or micronparticles; f: it is the water-soluble components and water-insoluble components of a cell or tissue components loaded together inside the nanoparticles or micronparticles, while the water-soluble components of a cell or tissue components alone are loaded on the surface of the nanoparticles or micronparticles; g: it is the water-soluble components and water-insoluble components of a cell or tissue components loaded together inside the nanoparticles or micronparticles, while the water-insoluble components of a cell or tissue components alone are loaded on the surface of the nanoparticles or micronparticles; h: the water-insoluble components in a cell or tissue components are loaded inside the nanoparticles or micronparticles alone, while the water-soluble components and water-insoluble components in a cell or tissue components are together loaded on the surface of the nanoparticles or micronparticles; i: water-soluble components in a cell or tissue components alone are loaded inside the nanoparticles or micronparticles, while the water-soluble components and water-insoluble components in a cell or tissue components are loaded together on the surface of nanoparticles or micronparticles.

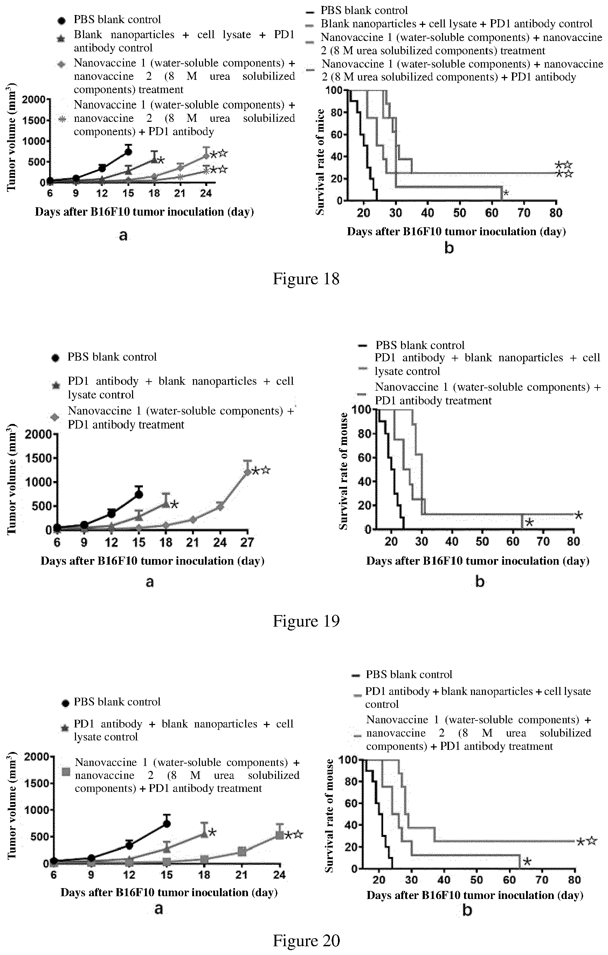

Figures 18-21 are the experimental results of the effects of the nanovaccine in Examples 1-3 on the tumor growth and survival of mice when the nanovaccine is used to treat melanoma; a, inhibitory effect of nanovaccine treatment on tumor growth (n≥8); b, survival effect experiment of nanovaccine treatment of mice inoculated with tumor (n≥8), each data point is presented as mean ± standard error (mean ± SEM); in panel a, the significant difference of the tumor growth inhibitory experiment was analyzed by ANOVA, and in panel b, the significant difference was analyzed by Kaplan-Meier and log-rank test; * indicates that compared with the PBS blank control group, the group has a p<0.05, which has a significant difference; ☆ means that compared with the blank nanoparticle + cell lysate + PD-1 antibody control group, this group has a p<0.05, which has a significant difference.

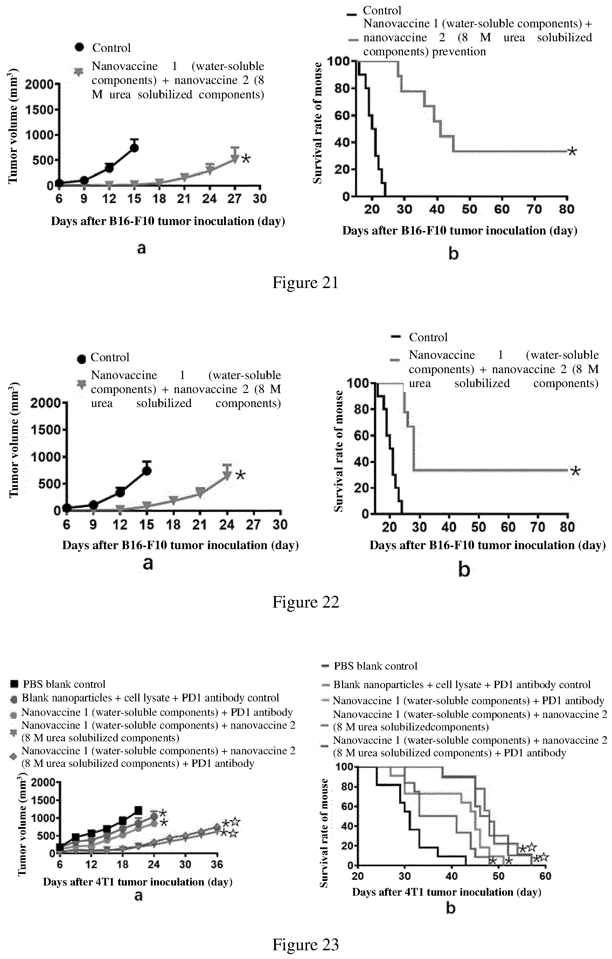

Figure 22 shows the experimental results of the effect on the tumor growth and survival of mice of the nanovaccines in Examples 4 and 5 when used to prevent melanoma; a, inhibitory effect experiment of nanovaccine treatment on tumor growth (n≥8); b, survival effect experiments of nanovaccine treatment on mice inoculated with tumors (n≥8), each data point is presented as mean ± standard error (mean ± SEM); in panel a, the significant difference of the tumor growth inhibitory experiment was analyzed by ANOVA, and in panel b, the significant difference was analyzed by Kaplan-Meier and log-rank test; * indicates that compared with the PBS blank control group, the group has a p<0.05, which has a significant difference.
Figures 23-28 are the experimental results of the effects of nanovaccines or micronvaccines in Examples 6-11 on the tumor growth and survival of mice when used to prevent or treat breast cancer; a, inhibitory effect experiment of nanovaccine or micronvaccine treatment on tumor growth (n≥7); b, survival effect experiment of the nanovaccine or micronvaccine treatment on mice inoculated with tumors (n≥7), each data point is presented as mean ± standard error (mean ± SEM); in panel a, the significant difference of the tumor growth inhibitory experiment was analyzed by ANOVA, and in panel b, the significant difference was analyzed by Kaplan-Meier and log-rank test; * indicates that compared with the PBS blank control group, the group has a p<0.05, which has a significant difference; ☆ means that compared with the blank nanoparticle + cell lysate + PD-1 antibody control group, this group has a p<0.05, which has a significant difference.

### DETAILED DESCRIPTION OF EMBODIMENTS

The present invention discloses a whole-cell components delivery system and application thereof. Those skilled in the art can learn from the content of this text and improve the process parameters appropriately to realize. It should be particularly pointed out that all similar substitutions and modifications are obvious to those skilled in the art, which are considered to be included in the present invention. The methods and products of the present invention have been described by preferred embodiments. Relevant persons can obviously make modifications or appropriate alternations and combinations of the methods described herein without departing from the content, spirit and scope of the present invention, so as to realize and apply the technology of the present invention.

In order to achieve the objective of the present invention, the technical solutions used in the present invention are as follows:

A whole-cell components delivery system consist of nano-sized or micron-sized particles and whole-cell components loaded on the particles, wherein the whole-cell components are water-soluble components and water-insoluble components of a whole cell in a cell or tissue.

In the present invention, the water-soluble components that are soluble in pure water or a water solution without a solubilizer are firstly collected after lysing cancer cells or tissues, and then the water-insoluble components are dissolved in a solubilizing solution with a solubilizing water solution containing a solubilizer. In this way, all cellular components can be converted into components that can be dissolved in a water solution to be loaded inside and on the surface of nanoparticles or micronparticles to prepare nanovaccine or micronvaccine for cancer prevention and treatment. In practice, the whole-cell components can also be dissolved in a solubilizing water solution containing a solubilizer directly after lysing cells or tissues without collecting water-soluble components and water-insoluble components respectively, and the whole-cell components dissolved in the solubilizing water solution are used to prepare nanovaccine or micronvaccine.

The present invention uses a water solution containing a solubilizer to convert the cellular components insoluble in pure water or a water solution without a solubilizer to that soluble in a specific solubilizing solution so as to be used to prepare nanoparticles and micronparticles. Thus, the fullness and immunogenicity of the antigen substances or components loaded on the nanoparticles or micronparticles are improved.

The whole-cell components in a cancer cell or tissue of the present invention is divided into water-soluble portion soluble in pure water or in a water solution without a solubilizer and water-insoluble portion which can be soluble in a water solution containing certain solubilizer. And the water-soluble portion and water-insoluble portion are loaded inside or on the surface of a nanoparticle or a micronparticle to ensure that most antigens are loaded in the vaccine prepared.

The water-soluble portion and the water-insoluble portion of the cell components include the components and constituents of an entire cell. Herein, the unmutated proteins, peptides and genes that have the same components with the normal cell components will not cause an immune response because of the immune tolerance generated during the development of the body immune system; and mutations in genes, proteins and peptides caused by cancer, etc. are immunogenic and can activate immune response because of lacking immune tolerance generated during the development of the body immune system. These immunogenic substances generated by mutations in disease in whole-cell components can be used for cancer prevention and treatment.

In order to enhance the immunogenicity and efficacy of the vaccine, certain immune enhancers with immune regulation function can also be added.

The whole-cell components delivery system described in the present invention can be used to prepare vaccines for preventing and/or treating cancer. The preparation process and application fields thereof are shown in Figure 1. During preparation, water-soluble components and water-insoluble components can be firstly collected respectively after lysation of a cell or tissue to prepare a nanovaccine or micronvaccine respectively; or the cell or tissue is lysed directly with a solubilizing solution containing a solubilizer, and the whole-cell components are dissolved in such solubilizing solution containing a solubilizer to prepare a nanovaccine or micronvaccine.

The whole-cell components of the present invention can be used to prepare a nanovaccine or micronvaccine after inactivation or (and) denaturation treatment before or (and) after lysation, or can be used directly to prepare a nanovaccine or micronvaccine without any inactivation or (and) denaturation treatment before or (and) after cell lysis. In some embodiments of the present invention, the tumor tissue cells are subject to inactivation or (and) denaturation treatment before lysis. In practice, the inactivation or (and) denaturation treatment can also be performed after cell lysis, or performed both before and after cell lysis. In some embodiments of the present invention, the inactivation or (and) denaturation treatment before or (and) after cell lysis are ultraviolet irradiation and high temperature heating. In practice, the inactivation or (and) denaturation treatment can also be radiation irradiation, high pressure, freeze drying and formaldehyde, etc. Those skilled in the art can understand that in practice, the skilled can make appropriate adjustments according to specific conditions.

The schematic diagrams of the structure of the whole-cell components delivery system of the present invention are shown in Figure 2 to Figure 17. In practice, the system can be a nanoparticle or micronparticle with only a specific structure used, or a nanoparticle or micronparticle with two or more different structures used at the same time.

In embodiments, the immune enhancer is loaded inside a nanoparticle or micronparticle and together on the surface of the nanoparticle or micronparticle. In practice, the immune enhancer can also be loaded inside the nanoparticle or micronparticle alone, or on the surface of the nanoparticle or micronparticle alone, or no immune enhancer is added.

In some embodiments, as shown in Figure 2, the water-soluble portion soluble in pure water or (and) the water-insoluble portion solubilized by a solubilizer of cell components are firstly loaded inside a nanoparticle or micronparticle, and an immune enhancer is loaded together; then, the water-soluble portion or (and) the water-insoluble portion of the cell components are adsorbed on the surface of the nanoparticle, and the immune enhancer is adsorbed at the same time. In this way, the loading capacity of the cellular water-soluble components or water-insoluble components in the nanoparticle or micronparticle can be maximized. In practice, a cell or tissue can be lysed directly with a solubilizing solution containing a solubilizer (such as 8 M urea water solution or 6 M guanidine hydrochloride water solution) and the whole-cell components are dissolved in such solubilizing solution containing a solubilizer, and then a nanovaccine or micronvaccine is prepared.

The methods for preparing a nanovaccine and micronvaccine described in the present invention are common preparation methods. In some embodiments, the preparation of the nanovaccine adopts the double-emulsion method of the solvent evaporation method, the preparation material of the nanoparticle is organic polymer polylactic acid-glycolic acid copolymer (PLGA) with a molecular weight of 24KDa-38KDa, and the immune adjuvants are poly(I:C), Bacille Calmette-Guerin (BCG) or CpG. Those skilled in the art can understand that in practice, the skilled can appropriately adjust the preparation method, preparation process, preparation materials for a nanoparticle, types and concentrations of the immune adjuvants, etc. according to specific condition.

In some embodiments, the specific preparation method of the double-emulsion method used in the present invention is as follows:
Step 1, a water phase solution with the first predetermined volume and the first predetermined concentration to an organic phase with the second predetermined volume and the second predetermined concentration of a medical polymer material.

In some embodiments, the water phase solution contains each component in a cancer cell lysate and an immune enhancing adjuvant, poly(I:C), BCG or CpG; each component in the cancer cell lysate were water-soluble component or original water-insoluble component dissolved in 8 M urea during preparation, respectively. The concentration of the water-soluble components from cancer cells or the original water-insoluble components from cancer cells dissolved in 8M urea contained in the water phase solution, that is, the first predetermined concentration, requires that the concentration of the protein peptide is greater than 1 ng/mL. The reason for such concentration is that through a large number of experiments, the inventors found that the higher the concentration of the water solution of cell lysate commonly used, the more the various types of cancer antigens are loaded in the prepared nanoparticles. When the prepared protein peptide has a concentration more than 1 ng/mL, that is, the first predetermined concentration is more than 1 ng/mL, enough cancer antigens can be loaded to activate relevant immune response. The concentration of the immune enhancing adjuvant in the initial water phase was more than 0.01 ng/mL. Additionally, the first predetermined volume is 600 µL.

In some embodiments, the water phase solution contains each component in a cancer tissue lysate and an immune enhancing adjuvant poly(I:C), BCG or CpG; each component in the cancer tissue lysate were water-soluble component or original water-insoluble component dissolved in 8 M urea during preparation, respectively. The concentration of the water-soluble components from cancer tissues or the original water-insoluble components from cancer tissues dissolved in 8M urea contained in the water phase solution, that is, the first predetermined concentration, requires that the concentration of the protein polypeptide is more than 1 ng/mL. The reason for such concentration is that through a large number of experiments, the inventors found that the higher the concentration of the water solution of tissue lysate commonly used, the more the various types of cancer antigens are loaded in the prepared nanoparticles. When the prepared protein polypeptide has a concentration more than 1 ng/mL, that is, the first predetermined concentration is more than 1 ng/mL, enough cancer antigens can be loaded to activate relevant immune response. The concentration of the immune enhancing adjuvant in the initial water phase is more than 0.01 ng/mL. Additionally, the first predetermined volume is 600 µL.

In the present invention, the organic phase containing the medical polymer material with the second predetermined volume and the second predetermined concentration is obtained by dissolving the medical polymer material in the organic solvent. In some embodiments, the medical polymer material is PLGA, the organic solvent is dichloromethane, and the volume of the obtained organic phase, that is, the second predetermined volume is 2 mL. In addition, in some embodiments, the range of the second predetermined concentration of the medical polymer material is 0.5 mg/mL-5000 mg/mL, preferably 100 mg/mL.

In the present invention, PLGA was chosen because it is a biodegradable material and has been approved by the FDA for use as a pharmaceutical adjuvant. Studies have shown that PLGA has certain immunomodulatory functions, so it is suitable to be used as an adjuvant for vaccine preparation.

In practice, the second predetermined volume of the organic phase is set according to its ratio to the first predetermined volume of the water phase. In the present invention, the range of the ratio of the first predetermined volume of the water phase to the second predetermined volume of the organic phase is 1:1.1 to 1:5000, preferably 1:10. In specific process, the first predetermined volume, the second predetermined volume and the ratio of the first predetermined volume to the second predetermined volume can be adjusted according to the need in order to adjust the size of the prepared nanoparticles.

Step 2, the mixed solution obtained in step 1 is subjected to ultrasonic treatment for more than 2 seconds or stirring for more than 1 minute.

This step is for nanometerization. The duration of the ultrasonic or the speed and duration of the stirring can control the size of the prepared nanoparticles, while the duration is too long or too short will result in the change of particle size. For this reason, it is necessary to choose appropriate ultrasonic duration. In the present invention, the duration of the ultrasonic is more than 2 seconds, the stirring speed is more than 50 rpm, and the stirring duration is more than 1 minute.

Step 3, the mixture obtained after the treatment in step 2 is added to an emulsifier water solution with the third predetermined volume and the third predetermined concentration, and subject to ultrasonic treatment for more than 2 seconds or stirring for more than 1 minute.

In this step, the mixture obtained in step 2 is added to the emulsifier water solution to continue ultrasonication or stirring for nanometerization.

In the present invention, the emulsifier water solution is a polyvinyl alcohol (PVA) water solution, the third predetermined volume is 5 mL, and the third predetermined concentration is 20 mg/mL. The third predetermined volume is adjusted according to its ratio to the second predetermined volume. In the present invention, the ratio of the second predetermined volume to the third predetermined volume is set in the range of 1:1.1 to 1:1000, preferably 2:5. In specific process, in order to control the size of the nanoparticles, the ratio of the second predetermined volume to the third predetermined volume can be adjusted.

Similarly, the duration of ultrasonication or stirring, the volume and concentration of the emulsifier water solution in this step are all valued in order to obtain nanoparticles of suitable size.

Step 4, the liquid obtained after the treatment in step 3 is added to an emulsifier water solution with the fourth predetermined volume and the fourth predetermined concentration, and subject to stirring until the predetermined stirring condition is satisfied.

In this step, the emulsifier water solution is still PVA, and the fourth predetermined volume is more than 50 mL, the fourth predetermined concentration is 5 mg/mL. The selection of the fourth predetermined concentration is based on obtaining nanoparticles with suitable size. The selection of the fourth predetermined volume is determined according to the ratio of the third predetermined volume to the fourth predetermined volume. In the present invention, the range of the ratio of the third predetermined volume to the third predetermined volume is 1:1.5-1:2000, preferably 1:10. In specific process, the ratio of the third predetermined volume to the fourth predetermined volume may be adjusted for controlling the size of the nanoparticles.

In the present invention, the predetermined stirring condition in this step is until the volatilization of the organic solvent is completed, that is, the volatilization of the dichloromethane in step 1 is completed.

Step 5, the mixed solution obtained in step 4 satisfying the predetermined stirring condition is centrifuged for more than 1 minute at a rotating speed more than 100 RPM. The supernatant was removed and the remaining precipitation was resuspended in a water solution containing a lyoprotectant with the fifth predetermined volume and the fifth predetermined concentration or PBS (or saline solution) with the sixth predetermined volume.

In some embodiments of the present invention, the precipitation obtained in step 5 does not need to be lyophilized when resuspended in PBS (or saline solution) with the sixth predetermined volume, and can be subject directly to subsequent experiments related to the adsorption of cancer cell lysates on the surface of nanoparticles.

In some embodiments of the present invention, the precipitation obtained in step 5 needs to be freeze-drying when resuspended in the water solution containing a lyoprotectant, and can be subject to subsequent experiments related to the adsorption of cancer cell lysates on the surface of nanoparticles after freeze-drying.

In the present invention, the lyoprotectant is trehalose.

In the present invention, the fifth predetermined volume of the lyoprotectant in this step is 20 mL, and the fifth predetermined concentration is 4% by mass percentage. The reason for this setting is to avoid affecting the freeze-drying effect in subsequent freeze-drying.

Step 6, the suspension containing the lyoprotectant obtained in step 5 is subject to freeze-drying treatment, and the freeze-dried substance obtained is for later use.

Step 7, a nanovaccine or micronvaccine is obtained by mixing the nanoparticle-containing suspension obtained in step 5 by resuspending in PBS (or saline solution) with the sixth predetermined volume or the freeze-dried substance obtained in step 6 (containing nanoparticles and lyoprotectant after freeze-drying) resuspending with PBS (or saline solution) with the sixth predetermined volume, and the water-soluble components with the seventh predetermined volume or the original water-insoluble components dissolved in 8 M urea, and is standed for more than 1 minute.

In the present invention, the volume ratio of the sixth predetermined volume to the seventh predetermined volume is 1: 10000 to 10000:1, preferred volume ratio is 1: 100 to 100:1, and the most preferred volume ratio is 1:30 to 30:1.

In some embodiments, the volume of the suspension resuspending nanoparticles is 10 mL. The volume of the water-soluble components or the original water-insoluble components dissolved in 8M urea containing the cancer cell lysate or the tumor tissue lysate is 1 mL.

In the present invention, the water-soluble components or the original water-insoluble component dissolved in 8 M urea containing cancer cell lysate or tumor tissue lysate contains poly(I:C), Bacillus Calmette-Guerin (BCG) or CpG, and the concentration of poly(I:C), BCG, or CpG is more than 1 ng/mL.

The particle size of the nanovaccine or micronvaccine is nano-sized or micro-sized, which can ensure that the vaccine is phagocytosed by antigen-presenting cells. In order to improve the phagocytosis efficiency, the particle size should be within an appropriate range. The particle size of the nanovaccine is 1 nm-1000 nm, more preferably, the particle size is 30 nm-1000 nm, most preferably, the particle size is 100 nm-600 nm; the particle size of the micronvaccine is 1 µm-1000 µm, more preferably, the particle size is 1 µm-100 µm, more preferably, the particle size is 1 µm-10 µm, and most preferably, the particle size is 1 µm-5 µm. In this embodiment, the particle size of the nanoparticle vaccine is 100 nm-600 nm, and the particle size of the micronvaccine is 1 µm-5 µm.

In addition, in the present invention, urea and guanidine hydrochloride are used to solubilize the original water-insoluble components in cancer cell lysate or tumor tissue lysate, and in practice, any other solubilizing substances that can dissolve the original water-insoluble components in cancer cell lysates or tumor tissue lysates in water solution can be used, such as sodium deoxycholate, SDS, alkaline solution with a pH more than 7, acidic solution with a pH less than 7, albumin, lecithin, inorganic salts at high concentrations, Triton, Tween, DMSO, acetonitrile, ethanol, methanol, DMF, isopropanol, propanol, acetic acid, cholesterol, amino acids, glycosides, choline, Brij^{™}-35, Octaethylene glycol monododecyl ether, CHAPS, Digitonin, lauryldimethylamine oxide, IGEPAL^{®} CA-630; alternatively, the above solubilizing solution may be used to dissolve the water-soluble components and the water-insoluble component at the same time.

In addition, in the present invention, 8 M urea and 6 M guanidine hydrochloride water solution are used to solubilize the original water-insoluble components in the cancer cell lysate or the tumor tissue lysate, and in practice, any other concentration of urea or guanidine hydrochloride that can dissolve the original water-insoluble components in the cancer cell lysate or the tumor tissue lysate in water solution can also be used; or 8M urea water solution is used to dissolve water-soluble components and water-insoluble components at the same time.

In addition, in the present invention, the double-emulsion method is used for preparation of the nanovaccine, and in practice, any other commonly preparation method of nanoparticle or micronparticle can also be used.

In addition, in the present invention, the preparation material of the nanovaccine is PLGA, and in practice, any other material that can also be used to prepare nanoparticles or micronparticles.

In addition, in the present invention, the water-soluble components or the original water-insoluble components dissolved in 8 M urea in the cancer cell lysate or the tumor tissue lysate is respectively loaded inside the nanoparticle and adsorbed on the surface of the nanoparticle, and in practice, the water-soluble components and the original water-insoluble components dissolved in 8 M urea in the cancer cell lysate or the tumor tissue lysate can also be mixed and then loaded inside the nanoparticle or adsorbed to the surface of the nanoparticle; alternatively, the water-soluble components and the water-insoluble components can be dissolved in 8 M urea at the same time and then loaded inside the nanoparticle or micronparticle and/or adsorbed on the surface of the nanoparticle or micronparticle.

In addition, in the present invention, poly(I:C), Bacille Calmette-Guérin (BCG) and CpG are used as immune adjuvants, and in practice, no immune adjuvant or any other immune adjuvant with immune enhancing function can be added, such as pattern recognition receptor agonist, residues frommethanol extraction of BCG, BCG cell wall acyl dipeptide, *Mycobacterium phlei*, polyactin A, mineral oil, virus-like particles, immune enhanced reconstituted influenza virus body, cholera enterotoxin, saponin and derivatives thereof, Resiquimod, thymosin, newborn bovine liver active peptides, miquimod, polysaccharides, curcumin, immune adjuvant poly ICLC, *Corynebacterium parvum* bacterin, hemolytic streptococcus preparation, coenzyme Q10, levamisole, polyinosinic acid, interleukin, interferon, polyinosinic acid, polyadenylic acid, alum, aluminum phosphate, lanolin, vegetable oil, endotoxin, liposome adjuvants, GM-CSF, MF59, double-stranded RNA, double strand DNA, aluminum hydroxide, CAF01, ginseng, active ingredients of traditional Chinese medicine such as Astragalus membranaceus etc..

In addition, in the present invention, the vaccine used in some embodiments is a nanovaccine, and in some embodiments is a micronvaccine. Those skilled in the art can choose to use a nanovaccine or micronvaccine according to actual situation.

In order to further understand the present invention, the technical solutions in the embodiments of the present invention will be described clearly and completely below in accompany with the examples of the present invention. Obviously, the described examples are only some, but not all, examples of the present invention. Based on the examples of the present invention, all other examples obtained by those of ordinary skilled in the art without creative efforts shall fall within the protection scope of the present invention.

Otherwise specified, the methods used in the examples of the present invention are conventional methods; the materials, reagents, etc. used are commercially available. The structure of a nano-sized particle or micro-sized particle, preparation method, utilization strategy in disease treatment, combination strategy with other immunotherapeutic drugs, combination strategy with other targeting therapeutic drugs, etc. involved in the examples of the present invention are only representative methods, and other structures of a nano-sized particle or micro-sized particle, preparation methods, utilization strategies in disease treatment, combination strategies with other immunotherapeutic drugs, and combination strategies with other targeting therapeutic drugs can also use the methods described in the present invention. The examples only list the application of the present invention in some cancers, but the present invention can also be used in other cancers. For the specific methods or materials used in the examples, those skilled in the art can make conventional substitution selections based on the technical ideas of the present invention and existing technologies, and are not limited to the specific descriptions of the examples of the present invention.

PD-1 antibody has been approved for the treatment of many cancers due to its excellent clinical efficacy. Therefore, in the examples of the present invention, the applicant also tested the combination of a nanovaccine or micronvaccine with PD-1 antibody to treat cancer in addition to using the nanovaccine or micronvaccine alone to treat cancer. In practice, the specific administration time, administration frequency, dosage regimen, and combination with other drugs can be adjusted according to the situation.

### Example 1 Whole-cell components of melanoma cancer cell loaded inside and on the surface of nanoparticles for cancer treatment

This example uses mouse melanoma as a cancer model to illustrate how to prepare a nanovaccine loaded with whole-cell components and apply this vaccine to treat melanoma.

PD-1 antibodies are approved for the treatment of many cancers due to their excellent clinical efficacy. Therefore, in this example, the applicant has tested the combination of a nanovaccine with a PD-1 antibody for the treatment of melanoma in addition to the nanovaccine described above for the treatment of melanoma only. The detailed administration time, administration frequency and dosage regimen can be adjusted with practice in application.

In this example, B 16F10 mouse melanoma cells were used as a cancer cell model. B 16F10 was firstly lysed to prepare water-soluble components and water-insoluble components of B16F10 cells. A nanovaccine loaded with the water-soluble and water-insoluble components of B16F10 cells was then prepared using organic polymer material PLGA as the backbone of nanoparticles and polyinosinic-polycytidylic acid (poly(I:C)) as the immune adjuvant by solvent volatilization method. The nanovaccines were then used to treat tumors in B16F10 melanoma-bearing mice.

### (1) Lysis of cancer cells and collection of each components

A certain amount of B16F10 cells were collected and frozen at -20°C to -273°C after removal of medium. The cells were subject to freeze thawing for at least 3 times with addition of certain amount of ultrapure water, which can be accompanied by ultrasound to destroy the lysed cells. After cell lysis, the lysate was centrifuged at more than 100g for at least 1 minute and the supernatant was taken to be the water-soluble components of B16F10. 8 M of urea was added into the resulting precipitated portion to dissolve the precipitated portion, so as to change the water-insoluble components of B16F10 in pure water to be soluble in 8 M of urea solution. The water-soluble components derived from the cancer cell lysate and the original water-insoluble components dissolved in 8 M of urea were the source of raw materials for the preparation of a nanovaccine for the treatment of cancer.

### (2) Preparation of nanovaccines

In this example, the nanovaccine and the blank nanoparticles as control were prepared using the double emulsion method of the solvent volatilization method. The nanoparticles were prepared using PLGA with a molecular weight of 24KDa-38KDa. The applied immune adjuvant is poly(I:C) and poly(I:C) is distributed both inside and on the surface of the nanoparticles. The preparation method was as described above. The average particle size of the nanoparticles was about 250nm before the loading of cellular components and immune adjuvant on the nanoparticle surface, while the average particle size of the nanovaccine was about 300 nm after the adsorption of cellular components and immune adjuvant on the nanoparticle surface, and the Zeta potential of the nanoparticle surface was about -5mV. About 150µg protein or peptide component was loaded on each 1 mg of PLGA nanoparticles, and the total amount of immune adjuvant poly(I:C) used inside and outside is approximately 0.01 mg, and half inside and half outside. The particle size of the blank nanoparticles was approximately 215 nm. The blank nanoparticles were prepared using pure water or 8 M urea containing equal amount of poly(I:C) in replacement of corresponding water-soluble and water-insoluble components, respectively. The blank nanoparticles were adsorbed with the same amount of poly(I:C) as that with nanovaccine on the external surface.

### (3) Nanovaccines for cancer treatment

In this study, two different administration methods were studied respectively: nanovaccine alone; nanovaccine in combination with PD-1 antibody. The control groups were the PBS group and the PD-1 antibody+blank nanoparticle+cell lysate group, respectively.

Female C57BL/6 of 6-8 weeks were selected as model mice for the preparation of melanoma-bearing mice.

The dosage regimen for nanovaccine alone group was as follows: 150,000 B16F10 cells were inoculated subcutaneously on the lower right side of the back of each mouse on day 0, and 200 µL of 2 mg PLGA nanoparticles loaded with the water-soluble components of the cancer cell lysate both inside and on the surface, and 200 µL of 2 mg PLGA nanoparticles loaded with the original water-insoluble components dissolved in 8 M urea both inside and on the surface were subcutaneously administered on day 4, day 7, day 10, day 15 and day 20, respectively.

The dosage regimen for nanovaccine in combination with PD-1 antibody was as follows: 150,000 B16F10 cells were inoculated subcutaneously on the lower right side of the back of each mouse on day 0, and 200 µL of 2 mg PLGA nanoparticles loaded with the water-soluble components of the cancer cell lysate both inside and on the surface, and 200 µL of 2mg of PLGA nanoparticles loaded with the original water-insoluble component dissolved in 8 M urea both inside and on the surface were subcutaneously administered on day 4, day 7, day 10, day 15 and day 20, respectively. PD-1 antibody was injected intraperitoneally on day 3, day 6, day 9 and day 14 with a dose of 10 mg/kg per mouse.

The dosage regimen of the PBS blank control group was as follows: 150,000 B16F10 cells were inoculated subcutaneously on the lower right side of the back of each mouse on day 0, and 400 µL of PBS was injected subcutaneously on day 4, day 7, day 10, day 15 and day 20.

PD-1 antibody+blank nanoparticle+cell lysate control group: 150,000 B16F10 cells were inoculated subcutaneously on the lower right side of the back of each mouse on day 0. Equal amounts of the water-soluble component of the cancer cell lysate, equal amounts of the original water-insoluble component of the cancer cell lysate dissolved in 8M urea and 4 mg of PLGA blank nanoparticles loaded with equal amounts of poly(I:C) without any cell lysate component were injected subcutaneously on day 4, day 7, day 10, day 15 and day 20, respectively. It should be noted that the three components need to be injected separately and injected at different sites to avoid adsorption of free cell lysate to the surface of the blank nanoparticles. PD-1 antibody was injected intraperitoneally on day 3, day 6, day 9 and day 14, respectively, with a dose of 10 mg/kg per mouse.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 18, compared with the PBS blank control group, the tumor growth rate of mice in the control blank nanoparticle+cell lysate+PD1 antibody group was significantly lower (p<0.05) and the survival of mice was significantly prolonged (p<0.05). Furthermore, compared with the PBS blank control group and blank nanoparticle+cell lysate+PD1 antibody control group, tumor growth rate was significantly lower in the nanovaccine alone group and nanovaccine+PD1 antibody combination group (p<0.05) and the survival of mice was significantly prolonged (p<0.05). Furthermore, 25% of the mice in both administration groups were cured and the tumor completely disappeared. When the dosage regimen for nanovaccine in combination with PD1 antibody is used in this example, the synergistic effect of the two was not significant in prolonging the survival of the mice.

In summary, the nanovaccine loaded with water-soluble components and water-insoluble components of cancer cells described in the present disclosure has therapeutic effect on melanoma and could partially cure melanoma.

### Example 2 Water-soluble cell components of melanoma cancer cells loaded inside and on the surface of nanoparticles for cancer treatment

This example uses mouse melanoma as a cancer model to illustrate how to prepare a nanovaccine loaded with the water-soluble portion of the cell components alone and apply the vaccine to treat melanoma.

PD-1 antibodies are approved for the treatment of many cancers due to their excellent clinical efficacy. Therefore, in this example, the applicant has tested the combination of the nanovaccine with a PD-1 antibody to treat melanoma cancer. In the application in practice, only the original water-insoluble portion of cell components can also be applied to prepare a nanovaccine to treat cancer. The detailed administration time, administration frequency and dosage regimen can be adjusted with practice in application.

In this embodiment, B16F10 mouse melanoma cells were used as a cancer cell model. B16F10 was firstly lysed to prepare water-soluble components and water-insoluble components of B16F10 cells. A nanovaccine loaded with the water-soluble components of B16F10 cells was then prepared using organic polymer material PLGA as the backbone of nanoparticles and poly(I:C) as the immune adjuvant by solvent volatilization method. The nanovaccines were then used to treat tumors in melanoma-bearing mice.

### (1) Lysis of cancer cells and collection of each components

A certain amount of B16F10 cells were collected and frozen at -20°C to -273°C after removal of medium. The cells were subject to freeze thawing for at least 3 times with addition of certain amount of ultrapure water, which can be accompanied by ultrasound to destroy the lysed cells. After cell lysis, the lysate was centrifuged at more than 100g for at least 5 minutes and the supernatant was taken to be the water-soluble components dissolved in pure water of B16F10. The water-soluble components derived from the cancer cell lysate was the source of raw material for the preparation of a nanovaccine for the treatment of cancer.

### (2) Preparation of nanovaccines

In this example, the nanovaccine and the control blank nanoparticles were prepared using the double emulsion method of the solvent volatilization method. The nanoparticles were prepared using PLGA with a molecular weight of 24KDa-38KDa and poly(I:C) as the immune adjuvant, with poly(I:C) being distributed both inside and on the surface of the nanoparticles. The preparation method was as described above. The average particle size of the nanoparticles was about 250nm before the loading of cellular component and immune adjuvant on the nanoparticle surface, while the average particle size of the nanovaccine was about 300 nm after the adsorption of cellular components and immune adjuvant on the nanoparticle surface, and the average Zeta potential of the nanoparticle surface was about -5mV. About 150µg protein or peptide components was loaded on each 1 mg of PLGA nanoparticles, and the total amount of immune adjuvant poly(I:C) used inside and outside is approximately 0.01 mg, and half inside and half outside. The particle size of the blank nanoparticles was approximately 215 nm. The blank nanoparticles were prepared using pure water or 8 M urea containing equal amount of poly(I:C) in replacement of corresponding water-soluble and water-insoluble components, respectively. The blank nanoparticles were adsorbed with the same amount of poly(I:C) as that with nanovaccine on the external surface.

### (3) Nanovaccines for cancer treatment

The study applied water-soluble components of nanovaccine alone in combination with PD-1 antibody to treat cancer.

Female C57BL/6 of 6-8 weeks were selected as model mice for the preparation of melanoma-bearing mice.

The dosage regimen for nanovaccine in combination with PD-1 antibody group was as follows: 150,000 B16F10 cells were inoculated subcutaneously on the lower right side of the back of each mouse on day 0. 200 µL of 2 mg PLGA nanovaccines loaded with the water-soluble components of the cancer cell lysate both inside and on the surface, and 200 µL of 2 mg PLGA blank nanoparticles loaded with equal amount of poly(I:C) and water-insoluble components (has equal amount of poly(I:C)) dissolved in 8 M urea from cell lysate, the three components were subcutaneously injected at different sites on day 4, day 7, day 10, day 15 and day 20, respectively. PD-1 antibody was injected intraperitoneally on day 3, day 6, day 9, and day 14, respectively, with a dose of 10 mg/kg per mouse.

The dosage regimen for PBS blank control group was as follows: 150,000 B16F10 cells were inoculated subcutaneously on the lower right side of the back of each mouse on day 0, and 400 µL of PBS was injected subcutaneously on day 4, day 7, day 10, day 15 and day 20.

PD-1 antibody+blank nanoparticle+cell lysate control group: 150,000 B16F10 cells were inoculated subcutaneously on the lower right side of the back of each mouse on day 0. 200 µL of equal amounts of the water-soluble component of the cancer cell lysate, 200 µL of equal amounts of the original water-insoluble component of the cancer cell lysate dissolved in 8 M urea and 4 mg of PLGA blank nanoparticles loaded with equal amounts of poly(I:C) without any cell lysate component were injected subcutaneously on day 4, day 7, day 10, day 15 and day 20, respectively. It should be noted that the three components need to be inoculated separately and injected at different sites to avoid adsorption of free cell lysate to the surface of the blank nanoparticles. PD-1 antibody was injected intraperitoneally on day 3, day 6, day 9 and day 14, respectively, with a dose of 10 mg/kg per mouse.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 19, compared with the PBS blank control group, the tumor growth rate of mice in the blank nanoparticle+cell lysate+PD1 antibody control group and nanovaccine administration group was significantly lower (p<0.05) and the survival of mice was significantly longer (p<0.05). In addition, compared with the blank nanoparticle+cell lysate+PD1 antibody control group, the tumor growth rate of mice in the nanovaccine administration group was significantly lower (p<0.05). Moreover, 12.5% of the mice in nanovaccine administration group were cured and the tumor completely disappeared. In summary, the nanovaccine loaded with water-soluble components of cancer cells described in the present disclosure has therapeutic effect on melanoma and could partially cure melanoma.

### Example 3 Lysis components of melanoma tumor tissue loaded inside and on the surface of nanoparticles for cancer treatment

This example uses mouse melanoma to illustrate how to prepare a nanovaccine loaded with lysis components of melanoma tumor tissue and apply the vaccine to treat melanoma.

In this example, the applicant has tested the effect of the nanovaccine in combination with a PD-1 antibody for the treatment of melanoma, the nanovaccine alone could also be used to treat cancer in application in practice.

In this embodiment, the lysis components of mouse melanoma tumor tissue were loaded inside and on the surface of nanoparticles to prepare nanovaccines. Mouse melanoma tumor tissue were firstly obtained and lysed to prepare water-soluble components and the original water-insoluble components dissolved in 8 M of urea. A nanovaccine loaded with the water-soluble components and water-insoluble components of tumor lysate was then prepared using PLGA as the backbone of nanoparticles and (poly(I:C)) as the immune adjuvant by solvent volatilization method. The nanovaccines were then used to treat tumors in melanoma-bearing mice.

### (1) Lysis of tumor tissue and collection of each component

150,000 B16F10 melanoma cells were inoculated subcutaneously on the back of each C57BL/6 mouse, the mouse was killed and its tumor tissue was extracted when the tumor inoculated in each mouse grew to a volume of approximately 80 mm³, 200 mm³, 400 mm³, 1000 mm³, respectively. Tumor tissue of the same size was cut into pieces and then ground, go through cell strainer and an appropriate amount of pure water was added to it and was subject to freeze thawing repeatedly for 5 times. After the lysis of the cells of tumor tissue, the cell lysate of tumor tissue was centrifuged at more than 12,000 RPM for 5 minutes, the supernatant was taken to be the water-soluble components of tumor tissue that could be dissolved in pure water. 8 M of urea was added into the resulting precipitated portion to dissolve the precipitated portion, so as to change the water-insoluble components of B16F10 in pure water to be soluble in 8 M of urea solution. The water-soluble components derived from the tumor tissue lysate and the original water-insoluble components dissolved in 8 M of urea was the source of raw materials for the preparation of a nanovaccine for the treatment of cancer cells.

### (2) Preparation of nanovaccines

In this example, the nanovaccine and the control blank nanoparticle were prepared using the double emulsion method of the solvent volatilization method. The nanoparticles were prepared using PLGA with a molecular weight of 24KDa-38KDa and poly(I:C) as the immune adjuvant, with poly(I:C) being distributed both inside and on the surface of the nanoparticles. The preparation method was as described above. The average particle size of the nanoparticles was about 250nm before the loading of cellular component and immune adjuvant on the nanoparticle surface, while the average particle size of the nanovaccine was about 300 nm after the adsorption of cellular component and immune adjuvant on the nanoparticle surface, and the average Zeta potential of the nanoparticle surface was about -5mV 160µg protein or peptide components was loaded on each 1 mg of PLGA nanoparticles, and the total amount of immune adjuvant poly(I:C) used inside and outside is approximately 0.01 mg, and half inside and half outside. The particle size of the blank nanoparticles was approximately 215 nm. The blank nanoparticles were prepared using pure water or 8 M urea containing an equal amount of poly(I:C) in replacement of corresponding water-soluble and water-insoluble components, respectively. The blank nanoparticles were adsorbed with the same amount of poly(I:C) as that with nanovaccine on the external surface.

### (3) Nanovaccines for cancer treatment

Female C57BL/6 of 6-8 weeks were selected as model mice for the preparation of melanoma-bearing mice.

The dosage regimen for nanovaccine group was as follows: 150,000 B16F10 cells were inoculated subcutaneously on the lower right side of the back of each mouse on day 0. 200 µL of 2 mg PLGA nanoparticles loaded with the water-soluble component of the tumor tissue lysate both inside and on the surface, and 200 µL of 2 mg PLGA nanoparticles loaded with the original water-insoluble components dissolved in 8 M urea both inside and on the surface were subcutaneously injected on day 4, day 7, day 10, day 15 and day 20, respectively. The tumor tissue lysate loaded by the nanovaccine injected on day 4 and day 7 was derived from the tumor mass of about 80 mm³ in size, while the tumor tissue lysate loaded by the nanovaccine injected on day 10 was derived from the tumor mass of about 200 mm³ in size, and the tumor tissue lysate loaded by the nanovaccine injected on day 15 was derived from the tumor mass of about 400 mm³ in size, and the tumor tissue lysate loaded by the nanovaccine injected on day 20 was derived from the tumor mass of about 1000 mm³ in size. PD-1 antibody was injected intraperitoneally on day 3, day 6, day 9, and day 14, respectively, with a dose of 10 mg/kg per mouse.

The dosage regimen for PBS blank control group was as follows: 150,000 B16F10 cells were inoculated subcutaneously on the lower right side of the back of each mouse on day 0, and 400 µL of PBS was injected subcutaneously on day 4, day 7, day 10, day 15 and day 20.

PD-1 antibody+blank nanoparticle+cell lysate control group: 150,000 B16F10 cells were inoculated subcutaneously on the lower right side of the back of each mouse on day 0. Equal amounts of the water-soluble component of the cancer cell lysate, equal amounts of the original water-insoluble component of the cancer cell lysate dissolved in 8M urea and 4 mg of PLGA blank nanoparticles loaded with equal amounts of poly(I:C) without any cell lysate component were injected subcutaneously on day 4, day 7, day 10, day 15 and day 20, respectively. It should be noted that the three components need to be injected separately and injected at different sites to avoid adsorption of free cell lysate to the surface of the blank nanoparticles. PD-1 antibody was injected intraperitoneally on day 3, day 6, day 9 and day 14, respectively, with a dose of 10 mg/kg per mouse.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 20, compared with the PBS blank control group and control blank nanoparticle+cell lysate+PD1 antibody, the tumor volume growth rate of mice in the nanovaccine administration group was significantly lower (p<0.05) and the survival of mice was significantly prolonged (p<0.05). Furthermore, 25% of the mice in nanovaccine administration group were cured and the tumor completely disappeared. In summary, the nanovaccine loaded with water-soluble components and water-insoluble components of tumor tissue lysate described in the present disclosure has therapeutic effect on melanoma and could partially cure melanoma.

### Example 4 Whole-cell components of melanoma cancer cells loaded inside and on the surface of nanoparticles for cancer prevention

This example uses mouse melanoma as a cancer model to illustrate how to prepare a nanovaccine loaded with whole-cell components and apply this vaccine to prevent melanoma.

In this embodiment, B16F10 mouse melanoma cells were used as a cancer cell model. B16F10 was firstly lysed to prepare water-soluble components and water-insoluble components of B16F10 cells. A nanovaccine loaded with the water-soluble and water-insoluble components of B16F10 cells was then prepared using PLGA as the backbone of nanoparticles and poly(I:C) as the immune adjuvant by solvent volatilization method. The nanovaccines were then used to prevent melanoma.

### (1) Lysis of cancer cells and collection of each component

The method for lysis of cancer cells and collection of each component were the same to example 1.

### (2) Preparation of nanovaccines

In this example, the nanovaccine and the control blank nanoparticle were prepared using the double emulsion method of the solvent volatilization method. The nanoparticles were prepared using PLGA with a molecular weight of 24KDa-38KDa and poly(I:C) as the immune adjuvant, with poly(I:C) being distributed both inside and on the surface of the nanoparticles. The preparation method was as described above. The average particle size of the nanoparticles was about 250nm before the loading of cellular component and immune adjuvant on the nanoparticle surface, while the average particle size of the nanovaccine was about 300 nm after the adsorption of cellular component and immune adjuvant on the nanoparticle surface, and the average Zeta potential of the nanoparticle surface was about -5mV. About 150µg protein or peptide components was loaded on each 1 mg of PLGA nanoparticles, and the total amount of immune adjuvant poly(I:C) used inside and outside is approximately 0.01 mg, and half inside and half outside. The particle size of the blank nanoparticles was approximately 215 nm. The blank nanoparticles were prepared using pure water or 8 M urea containing poly(I:C) in replacement of corresponding water-soluble and water-insoluble components, respectively. The blank nanoparticles were adsorbed with the same amount of poly(I:C) as that with nanovaccine on the external surface.

### (3) Nanovaccines for cancer prevention

Female C57BL/6 of 6-8 weeks were selected as model mice for the preparation of melanoma-bearing mice. 200 µL of 2 mg PLGA nanovaccine loaded with the water-soluble component of the cancer cell lysate both inside and on the surface and 200 µL of 2 mg PLGA nanovaccine loaded with the original water-insoluble components dissolved in 8 M urea both inside and on the surface were subcutaneously injected on day 42, day 35, day 28, day 21 and day 14, respectively, before inoculating B16F10 cancer cells. 150,000 B16F10 cells were inoculated subcutaneously on the lower right side of the back of each mouse on 14 days after the final dose of nanovaccine injection, and that day was set as day 0 of cancer cell inoculation.

In this experiment, the dosage regimen for the control group was as follows: 150,000 B16F10 cells were inoculated subcutaneously on the lower right side of the back of each mouse on day 0.

In the experiment, the size of the tumor volume of the mice was recorded every three days from day 6. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 21, compared with the control group, the tumor growth rate of mice in the nanovaccine prevention group was significantly lower (p<0.05) and the survival of mice was significantly prolonged (p<0.05). Additionally, the tumors disappeared in about 35% mice after tumor inoculation. In summary, the nanovaccine loaded with water-soluble components and water-insoluble components of cancer cell lysate described in the present disclosure has prevention effect on melanoma and could keep some inoculators away from melanoma.

### Example 5 Lysis components of melanoma tumor tissue loaded inside and on the surface of nanoparticles for cancer prevention

This example uses mouse melanoma as a cancer model to illustrate how to prepare a nanovaccine loaded with lysis components of melanoma tumor tissue and apply the vaccine to prevent melanoma.

In this embodiment, the lysis components of mouse melanoma tumor tissue were loaded inside and on the surface of nanoparticles to prepare nanovaccines. Mouse melanoma tumor masses were firstly obtained and lysed to prepare water-soluble components and the original water-insoluble components dissolved in 8 M of urea. A nanovaccine loaded with the water-soluble components and water-insoluble components of tumor lysate was then prepared using PLGA as the backbone of nanoparticles and poly(I:C) as the immune adjuvant by solvent volatilization method. The nanovaccines were then used to treat tumors in melanoma-bearing mice.

### (1) Lysis of tumor tissue and collection of each component

The preparation method for tumor tissue was the same to example 3.150,000 B16F10 melanoma cells were inoculated subcutaneously on the back of each C57BL/6 mouse, the mouse was killed and its tumor tissue was extracted when the tumor inoculated in each mouse grew to a volume of approximately 80 mm³, 200 mm³, 400 mm³, 1000 mm³, respectively. Tumor tissue of the same size was cut into pieces and then ground, go through a cell strainer and an appropriate amount of pure water was added to it, and then was subject to freeze thawing repeatedly for 3 times. After the lysis of the cells of tumor tissue, the cell lysate of tumor tissue was centrifuged at 12,000 RPM for 5 min, the supernatant was taken to be the water-soluble components of tumor tissue that could be dissolved in pure water. 8 M of urea was added into the resulting precipitated portion to dissolve the precipitated portion, so as to change the original water-insoluble components of B16F10 in pure water to be soluble in 8 M of urea solution. The water-soluble components and the original water-insoluble components derived from the tumor tissue lysate were the source of raw materials for the preparation of a nanovaccine for the prevention of melanoma.

### (2) Preparation of nanovaccines

In this example, the nanovaccine and the control blank nanoparticle were prepared using the double emulsion method of the solvent volatilization method. The nanoparticles were prepared using PLGA with a molecular weight of 24KDa-38KDa and poly(I:C) as the immune adjuvant, with poly(I:C) being distributed both inside and on the surface of the nanoparticles. The preparation method was as described above. The average particle size of the nanoparticles was about 250nm before the loading of cellular component and immune adjuvant on the nanoparticle surface, while the average particle size of the nanovaccine was about 300 nm after the adsorption of cellular component and immune adjuvant on the nanoparticle surface, and the average Zeta potential of the nanoparticle surface was about -5mV. 160µg protein or peptide components was loaded on each 1 mg of PLGA nanoparticles, and the total amount of immune adjuvant poly(I:C) used inside and outside is approximately 0.01 mg, and half inside and half outside. The particle size of the blank nanoparticles was approximately 215 nm. The blank nanoparticles were prepared using pure water or 8 M urea containing poly(I:C) in replacement of corresponding water-soluble and water-insoluble components, respectively. The blank nanoparticles were adsorbed with the same amount of poly(I:C) as that with nanovaccine on the external surface.

### (3) Nanovaccines for cancer prevention

Female C57BL/6 of 6-8 weeks were selected as model mice for the preparation of melanoma-bearing mice. 200 µL of 2 mg PLGA nanovaccine loaded with the water-soluble component both inside and on the surface and 200 µL of 2 mg PLGA nanovaccine loaded with the original water-insoluble components dissolved in 8 M urea both inside and on the surface were subcutaneously injected on day 42, day 35, day 28, day 21 and day 14, respectively, before inoculating B16F10 cancer cells. The tumor tissue lysate loaded by the nanovaccine injected on day 42 and day 35 before inoculating tumor was derived from the tumor mass of about 80 mm³ in size, while the tumor tissue lysate loaded by the nanovaccine injected on day 28 before inoculating tumor was derived from the tumor mass of about 200 mm³ in size, and the tumor tissue lysate loaded by the nanovaccine injected on day 21 before inoculating tumor was derived from the tumor mass of about 400 mm³ in size, and the tumor tissue lysate loaded by the nanovaccine injected on day 14 before inoculating tumor was derived from the tumor mass of about 1000 mm³ in size. 150,000 B16F10 cells were inoculated subcutaneously on the lower right side of the back of each mouse 14 days after the final dose of nanovaccine injection, and that day was set as day 0 of tumor inoculation.

In this experiment, the dosage regimen for the control group was as follows: 150,000 B16F10 cells were inoculated subcutaneously on the lower right side of the back of each mouse on day 0.

In the experiment, the size of the tumor volume of the mice was recorded every three days from day 6 after tumor inoculation. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 22, compared with the PBS blank control group, the tumor growth rate of mice in the nanovaccine prevention group was significantly lower (p<0.05) and the survival of mice was significantly prolonged (p<0.05). Additionally, the tumors disappeared in about 35% after tumor inoculation. In summary, the nanovaccine loaded with water-soluble components and original water-insoluble components of tumor tissue lysate described in the present disclosure has prevention effect on melanoma and could keep some inoculators away from melanoma.

### Example 6 Whole-cell components of breast cancer cells loaded inside and on the surface of nanoparticles for cancer treatment

This example uses mouse breast cancer treatment to illustrate how to prepare a nanovaccine loaded with whole-cell components and apply this vaccine to treat breast cancer.

In this example, the applicant has also tested the combination of a nanovaccine with a PD-1 antibody for the treatment of breast cancer in addition to applying the nanovaccine described above for the treatment of breast cancer only. The detailed administration time, administration frequency and dosage regimen can be adjusted with practice in application.

In this embodiment, 4T1 mouse triple-negative breast cancer cells were used as a cancer cell model. 4T1 was firstly lysed to prepare water-soluble components and water-insoluble components of 4T1 cells. A nanovaccine loaded with the water-soluble and water-insoluble components of 4T1 cells was then prepared using PLGA as the backbone of nanoparticles and poly(I:C) as the immune adjuvant by solvent volatilization method. The nanovaccines were then used to treat tumors in 4T1 breast-cancer-bearing mice.

### (1) Lysis of cancer cells and collection of each component

In this example, the methods for lysis of cancer cells, collection of lysates and solubilization of lysates were the same with example 1, except using 4T1 cells in replacement of B16F10 cells.

### (2) Preparation of nanovaccines

In this example, the methods for preparing nanovaccines and the materials used were the same with example 1, except using 4T1 cells in replacement of B16F10 cells.

### (3) Nanovaccines for cancer treatment

Female BALB/c of 6-8 weeks were selected as model mice for the preparation of tumor-bearing mice.

The dosage regimen for nanovaccine group was as follows: 400,000 4T1 cells were inoculated subcutaneously on the lower right side of the back of each mouse on day 0, and 200 µL of 2 mg PLGA nanoparticles loaded with the water-soluble component of the cancer cell lysate both inside and on the surface, and 200 µL of 2 mg PLGA nanoparticles loaded with the original water-insoluble components dissolved in 8 M urea both inside and on the surface were subcutaneously inoculated on day 4, day 7, day 10, day 15 and day 20, respectively.

The dosage regimen for nanovaccine in combination with PD-1 antibody group was as follows: 400,000 4T1 cells were inoculated subcutaneously on the lower right side of the back of each mouse on day 0, and 200 µL of 2 mg PLGA nanoparticles loaded with the water-soluble component both inside and on the surface, and 200 µL of 2mg of PLGA nanoparticles loaded with the original water-insoluble component dissolved in 8 M urea both inside and on the surface were subcutaneously administered on day 4, day 7, day 10, day 15 and day 20, respectively. PD-1 antibody was injected intraperitoneally on day 3, day 6, day 9 and day 14 with a dose of 10 mg/kg per mouse.

The dosage regimen for PBS blank control group was as follows: 400,000 4T1 cells were inoculated subcutaneously on the lower right side of the back of each mouse on day 0, and 400 µL of PBS was injected subcutaneously on day 4, day 7, day 10, day 15 and day 20, respectively.

PD-1 antibody+blank nanoparticle+cell lysate control group: 400,000 4T1 cells were inoculated subcutaneously on the lower right side of the back of each mouse on day 0. The water-soluble component of the cancer cell lysate, the original water-insoluble component of the cancer cell lysate dissolved in 8M urea and 4 mg of PLGA blank nanoparticles loaded with equal amounts of poly(I:C) without any cell lysate component were injected subcutaneously on day 4, day 7, day 10, day 15 and day 20, respectively. It should be noted that the three components need to be injected separately and injected at different sites to avoid adsorption of free cell lysate to the surface of the blank nanoparticles. PD-1 antibody was injected intraperitoneally on day 3, day 6, day 9 and day 14, respectively, with a dose of 10 mg/kg per mouse.

The dosage regimen for water-soluble components alone of nanovaccine in combination with PD-1 antibody was as follows: 150,000 B16F10 cells were inoculated subcutaneously on the lower right side of the back of each mouse on day 0. 200 µL of 2 mg PLGA nanovaccines loaded with the water-soluble component both inside and on the surface, 200 µL of 2 mg of PLGA blank nanoparticles loaded with equal amounts of poly(I:C) alone and water-insoluble component dissolved in 8 M urea were subcutaneously inoculated on day 4, day 7, day 10, day 15 and day 20, respectively. The three components were injected on different subcutaneous sites. PD-1 antibody was injected intraperitoneally on day 3, day 6, day 9 and day 14, respectively, with a dose of 10 mg/kg per mouse.

In the experiment, the size of the tumor volume of the mice was recorded every three days from day 6. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 23, compared with PBS blank control group and blank nanoparticle+cell lysate+PD1 antibody control group, the tumor growth rate of mice in the nanovaccine administration group was significantly lower (p<0.05) and the survival of mice was significantly prolonged (p<0.05). In summary, the nanovaccine loaded with water-soluble components and water-insoluble components of cancer cells described in the present disclosure has therapeutic effect on breast cancer.

When the dosage regimen for nanovaccine in combination with PD-1 antibody is used in this example, the synergistic effect of the two was not significant in prolonging the survival of the mice.

Compared with the blank nanoparticle+cell lysate+PD-1 antibody control group, the nanovaccine loaded with water-soluble components alone+PD-1 antibody group had no significant difference in terms of inhibiting the growth of tumor and prolonging the survival of the mice. It illustrated that using nanovaccine loaded with water-soluble components alone had no significant therapeutic effect to treat breast cancer.

### Example 7 Lysis components of breast cancer tumor tissue loaded inside and on the surface of nanoparticles for cancer treatment

This example uses mouse breast cancer as a cancer model to illustrate how to prepare a nanovaccine loaded with lysis components of breast cancer tumor tissue and apply the vaccine to treat breast cancer. The detailed administration time, administration frequency and dosage regimen can be adjusted with practice in application.

In this embodiment, the lysis components of mouse breast cancer tumor tissue were loaded inside and on the surface of nanoparticles to prepare nanovaccines. Mouse breast cancer tumor masses were firstly obtained and lysed to prepare water-soluble components and the original water-insoluble components dissolved in 8 M of urea. A nanovaccine loaded with the water-soluble components and water-insoluble components of tumor tissue lysate was then prepared using PLGA as the backbone of nanoparticles and poly(I:C) as the immune adjuvant by solvent volatilization method. The nanovaccines were then used to treat tumors in breast-cancer-bearing mice.

### (1) Lysis of cancer tissue and collection of each component

In this example, the method for lysis of cancer cells, collection of lysate and solubilization of lysate were the same to example 3, except using breast cancer tumor masses in replacement of melanoma tumor mass cells.

### (2) Preparation of nanovaccines

In this example, the method for preparing nanovaccines and the materials used were the same to example 3, except using 4 breast cancer tumor tissue in replacement of melanoma tumor tissue.

### (3) Nanovaccines for cancer treatment

Female BALB/c of 6-8 weeks were selected as model mice for the preparation of 4T1 tumor-bearing mice.

The dosage regimen for nanovaccine treatment group was as follows: 400,000 4T1 cells were inoculated subcutaneously on the lower right side of the back of each mouse on day 0, and 200 µL of 2 mg PLGA nanoparticles loaded with the water-soluble component both inside and on the surface, and 200 µL of 2 mg PLGA nanoparticles loaded with the original water-insoluble components dissolved in 8 M urea both inside and on the surface were subcutaneously administered on day 4, day 7, day 10, day 15 and day 20, respectively. The tumor tissue lysate loaded by the nanovaccine injected on day 4 and day 7 was derived from the tumor mass of about 80 mm³ in size, while the tumor tissue lysate loaded by the nanovaccine injected on day 10 was derived from the tumor mass of about 200 mm³ in size, and the tumor tissue lysate loaded by the nanovaccine injected on day 15 was derived from the tumor mass of about 400 mm³ in size, and the tumor tissue lysate loaded by the nanovaccine injected on day 20 was derived from the tumor mass of about 1000 mm³ in size.

The dosage regimen for PBS blank control group was as follows: 400,000 4T1 cells were inoculated subcutaneously on the lower right side of the back of each mouse on day 0, and 400 µL of PBS was inoculated subcutaneously on day 4, day 7, day 10, day 15 and day 20, respectively.

In the experiment, the size of the tumor volume of the mice was recorded every three days from day 6. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

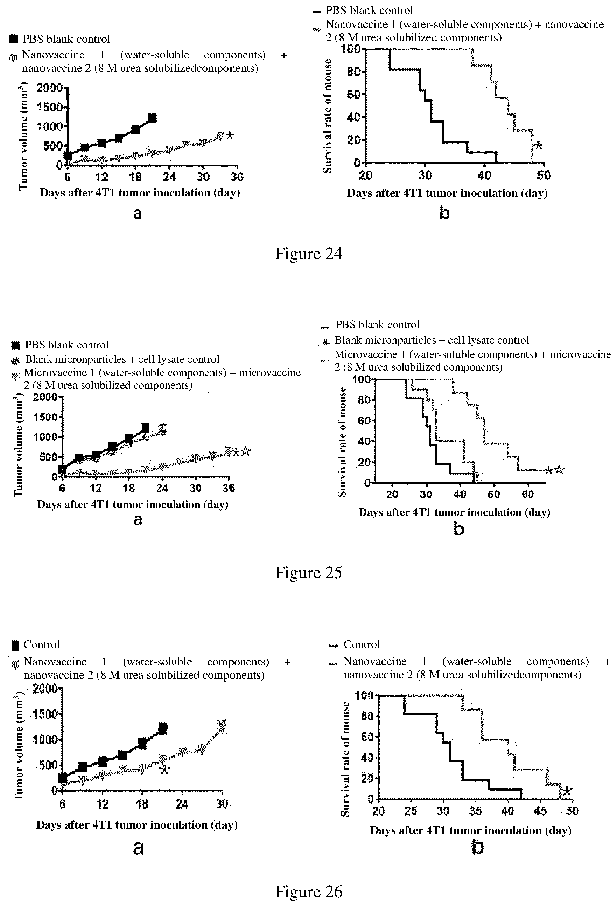

As shown in Figure 24, compared with PBS blank control group, the tumor growth rate of mice in the nanovaccine administration group was significantly lower (p<0.05) and the survival of mice was significantly longer (p<0.05). In summary, the nanovaccine loaded with water-soluble components and water-insoluble components of tumor tissue described in the present disclosure has therapeutic effect on breast cancer.

### Example 8 Whole-cell components of breast cancer cells loaded inside and on the surface of micronparticles for cancer treatment

This example uses mouse breast cancer as a cancer model to illustrate how to prepare a micronvaccine loaded with whole cell components and apply the vaccine to treat breast cancer.

In this embodiment, 4T1 mouse triple-negative breast cancer cells were used as a cancer cell model. 4T1 was firstly lysed to prepare water-soluble components and water-insoluble components of 4T1 cells. A micronvaccine loaded with the water-soluble and water-insoluble components of 4T1 cells was then prepared using PLGA as the backbone of micronparticles and poly(I:C) as the immune adjuvant by solvent volatilization method. The micronvaccines were then used to treat tumors in 4T1 breast-cancer-bearing mice.

### (1) Lysis of cancer cells and collection of each component

In this example, the method for lysis of cancer cells, collection of lysates and solubilization of lysate were the same to example 1, except using 4T1 cells in replacement of B16F10 cells.

### (2) Preparation of micronvaccines

In this example, the method for preparing micronvaccines and the materials used were the same to the example 1, except that the duration of ultrasonication of the primary emulsion and the secondary emulsion was shorter during the preparation of the micronparticles using the double-emulsion method. The average particle size of the micronvaccines prepared were about 2 µ m, the Zeta potential of the micronparticle surface was about -4mV. 200µg protein or peptide components was loaded on each 1 mg of PLGA micronparticles, and the total amount of immune adjuvant poly(I:C) used inside and outside each 1 mg of PLGA nanoparticle is approximately 0.01 mg, and half inside and half outside.

### (3) Micronvaccines for cancer treatment

Female BALB/c of 6-8 weeks were selected as model mice for the preparation of 4T1 tumor-bearing mice.

The dosage regimen for micronvaccine treatment group was as follows: 400,000 4T1 cells were inoculated subcutaneously on the lower right side of the back of each mouse on day 0, and 200 µL of 2 mg PLGA micronparticles loaded with the water-soluble component both inside and on the surface, and 200 µL of 2 mg PLGA micronparticles loaded with the original water-insoluble components dissolved in 8 M urea both inside and on the surface were subcutaneously injected on day 4, day 7, day 10, day 15 and day 20, respectively.

The dosage regimen for PBS blank control group was as follows: 400,000 4T1 cells were inoculated subcutaneously on the lower right side of the back of each mouse on day 0, and 400 µL of PBS was injected subcutaneously on day 4, day 7, day 10, day 15 and day 20, respectively.

Blank micronparticle+cell lysate control group: 400,000 4T1 cells were inoculated subcutaneously on the lower right side of the back of each mouse on day 0. Equal amounts of the water-soluble component of the cancer cell lysate, equal amounts of the water-soluble component of the cancer cell lysate, the original water-insoluble component of the cancer cell lysate dissolved in 8M urea and 4 mg of PLGA blank nanoparticles loaded with equal amounts of poly(I:C) without any cell lysate component were injected subcutaneously on day 4, day 7, day 10, day 15 and day 20, respectively. It should be noted that the three components need to be injected separately and injected at different sites to avoid adsorption of free cell lysate to the surface of the blank nanoparticles.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 25, compared with PBS blank control group and blank micronparticle control group, the tumor growth rate of mice in the micronvaccine administration group was significantly lower (p<0.05) and the survival of mice was significantly longer (p<0.05). In summary, the micronvaccine loaded with water-soluble components and water-insoluble components of cancer cells described in the present disclosure has therapeutic effect on breast cancer.

### Example 9 Lysis components of breast cancer tumor tissue loaded inside and on the surface of nanoparticles for cancer prevention

This example uses mouse breast cancer as a cancer model to illustrate how to prepare a nanovaccine loaded with lysis components of breast cancer tumor tissue and apply the vaccine to treat breast cancer.

In this embodiment, lysis components of mouse breast cancer tumor tissue were loaded inside and on the surface of nanoparticles to prepare nanovaccines. Mouse breast cancer tumor masses were firstly lysed to prepare water-soluble components and original water-insoluble components dissolved in 8 M urea of tumor mass tissue. A nanovaccine loaded with the water-soluble and water-insoluble components of cancer tissue lysate was then prepared using PLGA as the backbone of nanoparticles and poly(I:C) as the immune adjuvant by solvent volatilization. The nanovaccines were then used to treat tumors in breast-cancer-bearing mice.

### (1) Lysis of cancer tissue and collection of each component

In this example, the method for lysis of cancer tissue, collection of lysate and solubilization of lysate were the same to example 3, except using breast cancer tumor mass in replacement of melanoma tumor mass.

### (2) Preparation of nanovaccines

In this example, the method for preparing nanovaccines and the materials used were the same to example 3, except that the melanoma tumor mass was in replacement of breast cancer tumor mass. The average particle size of the nanoparticles was about 250nm before the loading of cellular component and immune adjuvant on the nanoparticle surface, while the average particle size of the nanovaccine was about 300 nm after the adsorption of cellular component and immune adjuvant on the nanoparticle surface. 150µg protein or peptide components was loaded on each 1 mg of PLGA nanoparticles, and the total amount of immune adjuvant poly(I:C) used inside and outside each 1 mg of PLGA nanoparticle is approximately 0.01 mg, and half inside and half outside. The Zeta potential of the nanoparticle surface was about -5mV.

### (3) Nanovaccines for cancer prevention

Female BALB/c of 6-8 weeks were selected as model mice for the preparation of 4T1 tumor-bearing mice.

The dosage regimen for nanovaccine prevention group: 200 µL of 2 mg PLGA nanovaccine loaded with the water-soluble component both inside and on the surface and 200 µL of 2 mg PLGA nanovaccine loaded with the original water-insoluble component dissolved in 8 M urea both inside and on the surface were subcutaneously injected on day 42, day 35, day 28, day 21 and day 14, before inoculating tumor cells, respectively. The tumor tissue lysate loaded by the nanovaccine injected on day 42 and day 35 before inoculating tumor was derived from the tumor mass of about 80 mm³ in size, while the tumor tissue lysate loaded by the nanovaccine injected on day 28 before inoculating tumor was derived from the tumor mass of about 200 mm³ in size, and the tumor tissue lysate loaded by the nanovaccine injected on day 21 before inoculating tumor was derived from the tumor mass of about 400 mm³ in size, and the tumor tissue lysate loaded by the nanovaccine injected on day 14 before inoculating tumor was derived from the tumor mass of about 1000 mm³ in size. 400,000 4T1 cells were inoculated subcutaneously on the lower right side of the back of each mouse 14 days after the final dose of nanovaccine injection, and that day was set as day 0.

The dosage regimen of the control group was as follows: 400,000 4T1 cells were inoculated subcutaneously on the lower right side of the back of each mouse on day 0.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 26, compared with the control group, the tumor growth rate of mice in the nanovaccine prevention group was significantly lower (p<0.05) and the survival of mice was significantly longer (p<0.05). In summary, the nanovaccine loaded with water-soluble components and water-insoluble components of breast cancer tumor mass tissue lysate described in the present disclosure has prevention effect on melanoma.

### Example 10 Whole-cell components loaded inside and on the surface of nanoparticles and Bacillus Calmette-Guérin (BCG) as the immune adjuvant for cancer treatment

This example uses mouse breast cancer as a cancer model and uses BCG as the immune adjuvant to illustrate how to prepare a nanovaccine loaded with whole-cell components and apply the vaccine to treat breast cancer.

In this embodiment, 4T1 mouse triple-negative breast cancer cells were used as a cancer cell model. 4T1 cells were firstly lysed to prepare water-soluble components and water-insoluble components of 4T1 cells. A nanovaccine loaded with the water-soluble and water-insoluble components of 4T1 cells was then prepared using PLGA as the backbone of nanoparticles and BCG as the immune adjuvant by solvent volatilization method. The nanovaccines were then used to treat tumors in 4T1 breast-cancer-bearing mice.

### (1) Lysis of cancer cells and collection of each component

In this example, the method for lysis of cancer cells, collection of lysate and solubilization of lysate were the same to example 1, except that using 4T1 cells in replacement of B16F10 cells.

### (2) Lysis of BCG and collection of each component

In this example, the method for lysis of BCG, collection of lysate and solubilization of lysate were the same to the method for lysis of cancer cells in example 1, except that using BCG in replacement of cancer cells.

### (3) Preparation of nanovaccines

In this example, the method for preparing nanovaccines and the materials used were the same to example 6, except that immune adjuvants loaded inside the nanovaccine were the water-soluble components and water-insoluble components of BCG instead of poly (I:C); besides, the immune adjuvants adsorbed on the surface of nanovaccine were BCG without lysis. The average particle size of the nanoparticles was about 250nm before the loading of cellular component and immune adjuvant on the nanoparticle surface, while the average particle size of the nanovaccine was about 310 nm after the adsorption of cellular component and immune adjuvant on the nanoparticle surface. 150µg protein or peptide component was loaded on each 1 mg of PLGA nanoparticles, and the total amount of immune adjuvant BCG used inside and outside each 1 mg of PLGA nanoparticle is approximately 0.1 mg, and half inside and half outside.

### (4) Nanovaccines for cancer treatment

Female BALB/c of 6-8 weeks were selected as model mice for the preparation of 4T1 tumor-bearing mice.

The dosage regimen for nanovaccine in combination with PD-1 antibody was as follows: 400,000 4T1 cells were inoculated subcutaneously on the lower right side of the back of each mouse on day 0, and 200 µL of 2 mg PLGA nanoparticles loaded with the water-soluble component both inside and on the surface, and 200 µL of 2mg of PLGA nanoparticles loaded with the original water-insoluble component dissolved in 8 M urea both inside and on the surface were subcutaneously injected on day 4, day 7, day 10, day 15 and day 20, respectively. PD-1 antibody was injected intraperitoneally on day 3, day 6, day 9 and day 14 with a dose of 10 mg/kg per mouse.

The dosage regimen for PBS blank control group was as follows: 400,000 4T1 cells were inoculated subcutaneously on the lower right side of the back of each mouse on day 0, and 400 µL of PBS was injected subcutaneously on day 4, day 7, day 10, day 15 and day 20, respectively.

PD-1 antibody+blank nanoparticle+cell lysate control group: 400,000 4T1 cells were inoculated subcutaneously on the lower right side of the back of each mouse on day 0. The water-soluble component of the cancer cell lysate, the original water-insoluble component of the cancer cell lysate dissolved in 8M urea and 4 mg of PLGA blank nanoparticles loaded with equal amounts of BCG without any cell lysate component were injected subcutaneously on day 4, day 7, day 10, day 15 and day 20, respectively. It should be noted that the three components need to be injected separately and injected at different sites to avoid adsorption of free cell lysate to the surface of the blank nanoparticles. PD-1 antibody was injected intraperitoneally on day 3, day 6, day 9 and day 14, respectively, with a dose of 10 mg/kg per mouse.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

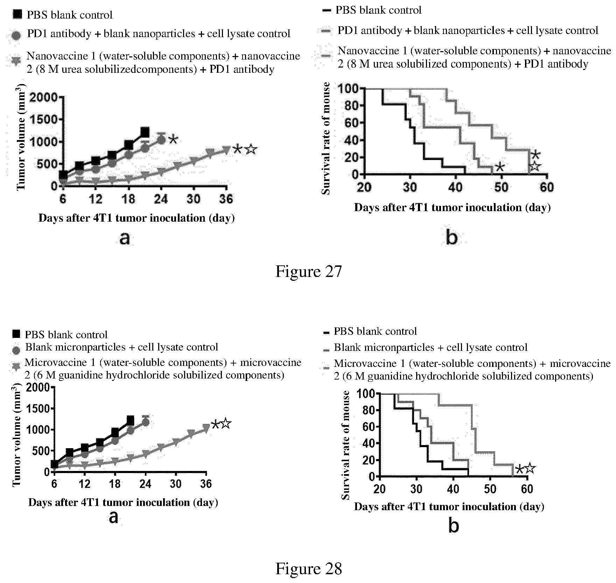

As shown in Figure 27, compared with the PBS blank control group and PD-1 antibody+blank nanoparticle+cell lysate control group, the tumor growth rate of mice in the administration group of nanovaccine with BCG as the immune adjuvant was significantly lower (p<0.05) and the survival of mice was significantly longer (p<0.05). In summary, the nanovaccine loaded with whole-cell components of cancer cells described in the present disclosure has therapeutic effect on breast cancer when using BCG as immune adjuvant.

### Example 11 6M guanidine hydrochloride dissolving tumor tissue components and loaded them inside and on the surface of nanoparticles for cancer treatment

This example uses mouse breast cancer as a cancer model to illustrate how to apply 6M guanidine hydrochloride to dissolve whole-cell components and prepare a nanovaccine loaded with whole-cell components to treat breast cancer. In this embodiment, 4T1 mouse triple-negative breast cancer cells were used as a cancer cell model. Tumor tissue cells were first inactivated, denatured, lysed with 6M guanidine hydrochloride and dissolves whole-cell components with 6M guanidine hydrochloride. A micronvaccine loaded with tumor tissue whole-cell components was then prepared using PLGA as the backbone material of the micronparticles, CpG as the immune adjuvant by solvent volatilization method. The micronvaccines were then used to treat tumors in 4T1 breast-cancer-bearing mice.

### (1) Lysis of cancer cells and collection of each component

400,000 4T1 breast cancer cells were inoculated subcutaneously on the back of each BALB/c mouse, the mouse was killed and its tumor tissue was extracted when the tumor inoculated in each mouse grew to a volume of approximately 80 mm³, 200 mm³, 400 mm³, 1000 mm³, respectively. Tumor tissue of the same size was cut into pieces and then ground, filtered through a cell strainer and the resulting tumor tissue cells were collected. The obtained tumor tissue cells were inactivated and denatured by ultraviolet light and high-temperature heating, respectively, and then an appropriate amount of 6M guanidine hydrochloride was applied to lyse tumor tissue cells and dissolve the tissue lysate, the resulting lysate was the source of raw materials for the preparation of a micronvaccine.

### (2) Preparation of micronvaccines

In this example, the method for preparing micronvaccines, blank micronparticles and materials used were the same to example 8, except that CpG was used as immune adjuvant. The average particle size of the micronvaccines prepared was about 2.5 µm, the Zeta potential of the micronparticle surface was about -4mV 210µg protein or peptide components was loaded inside and outside on the surface of each 1 mg of PLGA micronparticles, and the total amount of immune adjuvant CpG used inside and outside on the surface of each 1 mg of PLGA nanoparticle was approximately 0.01 mg, with half inside and half outside.

### (3) Micronvaccines for cancer treatment

Female BALB/c of 6-8 weeks were selected as model mice for the preparation of 4T1 tumor-bearing mice.

The dosage regimen for micronvaccine group was as follows: 400,000 4T1 cells were inoculated subcutaneously on the lower right side of the back of each mouse on day 0. 100 µL of 2 mg PLGA micronparticles loaded with the whole-cell components of the tumor tissue both inside and on the surface were subcutaneously injected on day 4, day 7, day 10, day 15 and day 20, respectively. The tumor tissue lysate loaded by the nanovaccine injected on day 4 and day 7 was derived from the tumor mass of about 80 mm³ in size, while the tumor tissue lysate loaded by the nanovaccine injected on day 10 was derived from the tumor mass of about 200 mm³ in size, and the tumor tissue lysate loaded by the nanovaccine injected on day 15 was derived from the tumor mass of about 400 mm³ in size, and the tumor tissue lysate loaded by the nanovaccine injected on day 20 was derived from the tumor mass of about 1000 mm³ in size.

The dosage regimen for PBS blank control group was as follows: 400,000 4T1 cells were inoculated subcutaneously on the lower right side of the back of each mouse on day 0, and100 µL of PBS was injected subcutaneously on day 4, day 7, day 10, day 15 and day 20, respectively.

Blank micronparticle+cell lysate control group: 400,000 4T1 cells were inoculated subcutaneously on the lower right side of the back of each mouse on day 0. Equal amounts of the cancer tissue lysate and 2 mg of PLGA blank micronparticles loaded with equal amounts of CpG without any cell lysate component were injected subcutaneously on day 4, day 7, day 10, day 15 and day 20, respectively. It should be noted that the two components need to be inoculated separately and injected at different sites to avoid adsorption of free cell lysate to the surface of the blank nanoparticles.

In the experiment, the size of the tumor volume of the mice was recorded every three days from day 6 onwards. The tumor volume was calculated using the formula v=0.52^{∗}a^{∗}b², wherein v is the tumor volume, a is the tumor length and b is the tumor width. For ethical reasons of animal experiment, a mouse was considered dead and euthanised when its tumor volume exceeded 2000 mm³ in the survival test.

### (4) Experimental results

As shown in Figure 28, compared with the PBS control group and blank micronparticle control group, the tumor growth rate of mice in the micronvaccine administration group was significantly lower (p<0.05) and the survival of mice was significantly longer (p<0.05). In summary, the micronvaccine loaded with whole-cell components of tumor tissue described in the present disclosure has therapeutic effect on breast cancer.

## Claims

1. A whole-cell components delivery system consisting of a nano-sized or micron-sized particle and whole-cell components loaded on the particle, wherein the whole-cell components are water-soluble components and water-insoluble components of a whole cell in a cell or tissue.

2. The delivery system of claim 1, wherein a form to load is: the water-soluble components and the water-insoluble components of the whole cell are separately or together loaded inside the particle, and/or, separately or together loaded on the surface of the particle.

3. The delivery system of claim 2, wherein the inside and/or the surface of the particle further comprises an immune enhancing adjuvant.

4. The delivery system of any one of claims 1-3, wherein the water-soluble components of a whole cell in a cell or tissue is original water-soluble portion of the whole cell in the cell or tissue which can be dissolved in pure water or water solution without a solubilizer; the water-insoluble components of the whole cell in the cell or tissue are original water-insoluble portion of the whole cell in the cell or tissue, wherein the original water-insoluble portion is converted from insoluble in pure water to soluble in water solution containing a solubilizer or organic solvent by suitable solubilization method.

5. The delivery system of claim 4, wherein the solubilizer is urea, guanidine hydrochloride, sodium deoxycholate, SDS, glycerol, alkaline solution with a pH more than 7, acidic solution with a pH less than 7, various protein degrading enzymes, albumin, lecithin, inorganic salts at high concentrations, Triton, DMSO, acetonitrile, ethanol, methanol, DMF, propanol, isopropanol, Tween, acetic acid, cholesterol, amino acids, glycosides, choline, Brij-35, Octaethylene glycol monododecyl ether, CHAPS, Digitonin, lauryldimethylamine oxide or IGEPAL^{®} CA-630; the organic solvent is DMSO, acetonitrile, ethanol, methanol, DMF, isopropanol, dichloromethane, propanol or ethyl acetate.

6. The delivery system of claim 3, wherein the immune enhancing adjuvant is at least one of immune enhancers derived from microorganism, products of human or animal immune systems, intrinsic immune agonists, adaptive immune agonists, chemically synthesized medicaments, fungal polysaccharides and traditional Chinese medicines.

7. The delivery system of claim 3, wherein the immune enhancing adjuvant is at least one of pattern recognition receptor agonists, Bacille Calmette-Guérin (BCG) vaccine, BCG cell wall skeleton, residues from methanol extraction of BCG, BCG cell wall acyl dipeptide, *Mycobacterium phlei*, thymosin, polyactin A, mineral oil, virus-like particles, immune enhanced reconstituted influenza virus bodies, cholera enterotoxin, saponin and derivatives thereof, Resiquimod, newborn bovine liver active peptides, miquimod, polysaccharides, curcumin, immune adjuvant CpG, immune adjuvant poly(I:C), immune adjuvant poly ICLC, *Corynebacterium parvum*, hemolytic streptococcus preparations, coenzyme Q10, levamisole, polyinosinic acid, interleukin, interferon, polyinosinic acid, polyadenylic acid, alum, aluminum phosphate, lanolin, vegetable oil, endotoxin, liposome adjuvants, GM-CSF, MF59, double-stranded RNA, double strand DNA, aluminum hydroxide, CAF01, active ingredients of ginseng and active ingredients of Astragalus membranaceus.

8. The delivery system of any one of claims 1-3, wherein the nano-sized particle have a particle size of 1 nm to 1,000 nm; the micron-sized particle have a particle size of 1 µm to 1,000 µm; the nano-sized particle and micron-sized particle are electrically neutral, negatively charged or positively charged on surface.

9. The delivery system of any one of claims 1-3, wherein the nano-sized or micron-sized particle is made of materials as organic synthetic polymer materials, natural polymer materials or inorganic materials.

10. The delivery system of claim 9, wherein the organic synthetic polymer materials are PLGA, PLA, PGA, PEG, PCL, Poloxamer, PVA, PVP, PEI, PTMC, polyanhydride, PDON, PPDO, PMMA, polyamino acid, synthetic peptides; the natural polymer materials are lecithin, cholesterol, sodium alginate, albumin, collagen, gelatin, cell membrane components, starch, sugar and polypeptides; and the inorganic materials are ferric oxide, ferric oxide, calcium carbonate, calcium phosphate.

11. The delivery system of any one of claims 1-3, wherein the delivery system is spherical, ellipsoidal, barrel-shaped, polygonal, rod-shaped, sheet-shaped, linear, worm-shaped, square, triangular, butterfly-shaped or disc-shaped.

12. A use of the whole-cell components delivery system of claim 1 in preparing vaccines for preventing and/or treating cancer.
